# EUROPEAN PATENT APPLICATION

(11) **EP 4 318 623 A1**
(43) Date of publication of application: **07.02.2024**
(21) Application number: 22774984.3
(22) Date of filing: 03.03.2022
(51) Int. Cl.: H01L 51/42, H01L 31/10, H01L 27/146, H01L 27/30

(54) **PHOTOELECTRIC CONVERSION ELEMENT AND IMAGING DEVICE**

(30) Priority: 23.03.2021 JP 2021048075
(71) Applicant: Panasonic Intellectual Property Management Co., Ltd., Osaka 571-0057 (JP)
(72) Inventor: KISHIMOTO, Yuko, Kadoma-shi, Osaka (JP); IIJIMA, Hiroaki, Kadoma-shi, Osaka (JP); HIRADE, Masaya, Kadoma-shi, Osaka (JP); IZUCHI, Masumi, Kadoma-shi, Osaka (JP)
(74) Representative: Grünecker Patent- und Rechtsanwälte PartG mbB
(86) International application number: PCT/JP2022/009124
(87) International publication number: WO 2022/202188

(57) **Abstract**

A photoelectric conversion device includes a first electrode, a second electrode facing the first electrode, and a photoelectric conversion layer located between the first electrode and the second electrode and including a bulk heterojunction layer containing a donor organic compound and an acceptor organic compound. The donor organic compound includes a first substituent. The acceptor organic compound includes an aromatic portion and a second substituent binding to the aromatic portion and having dipole-dipole interaction with the first substituent.

## Description

### Technical Field

The present disclosure relates to a photoelectric conversion device and an imaging apparatus.

### Background Art

Photoelectric conversion devices which convert light energy into electrical energy are broadly used as solar cells or optical sensors. For example, many photoelectric conversion devices using inorganic semiconductor materials such as silicon monocrystal or silicon polycrystal have been developed.

In addition, recently, for example, as described in NPL 1, organic semiconductor materials having physical properties and functions not found in conventional inorganic materials have been actively studied. Organic photoelectric conversion devices, which are photoelectric conversion devices using organic semiconductor materials as the photoelectric conversion materials of photoelectric conversion layers, have been also developed.

Photoelectric conversion devices can be used as optical sensors of imaging apparatuses and so on by extracting charges generated by light as electric signals.

### Citation List

### Non Patent Literature

NPL 1: JANA ZAUMSEIL et. al., "Electron and Ambipolar Transport in Organic Field-Effect Transistors", Chemical Reviews, American Chemical Society, 2007, Vol. 107, No. 4, pp. 1296-1323
NPL 2: YOSHIHIDE SANTO et. al., "Mixture of [60] and [70] PCBM giving morphological stability in organic solar cells", Applied Physics Letters, AIP Publishing, 2013, 103, 073306
NPL 3: Yoko Abe et. al., "Low-LUMO 56p-electron fullerene acceptors bearing electron-withdrawing cyano groups for small-molecule organic solar cells", Organic Electronics, Elsevier B.V., 2013, pp. 3306-3311

### Summary of Invention

### Technical Problem

A photoelectric conversion layer using an organic semiconductor material of which molecules are aggregated by weak van der Waals force, unlike inorganic semiconductors which bind by strong covalent bonds, has a problem that the photoelectric conversion layer is degenerated and the device characteristics deteriorate when the layer is exposed to high temperature by, for example, heating to high temperature, after formation.

Accordingly, the present disclosure provides a photoelectric conversion device or the like that can suppress the deterioration of the device characteristics when exposed to high temperature.

### Solution to Problem

A photoelectric conversion device according to one aspect of the present disclosure includes a first electrode, a second electrode facing the first electrode, and a photoelectric conversion layer located between the first electrode and the second electrode and including a bulk heterojunction layer containing a donor organic compound and an acceptor organic compound. The donor organic compound includes a first substituent. The acceptor organic compound includes an aromatic portion and a second substituent binding to the aromatic portion and having dipole-dipole interaction with the first substituent.

The imaging apparatus according to one aspect of the present disclosure includes a substrate and a pixel including a charge detection circuit provided in the substrate, a photoelectric conversion unit provided on the substrate, a charge storage node electrically connected to the charge detection circuit and the photoelectric conversion unit. The photoelectric conversion unit includes the above-described photoelectric conversion device.

### Advantageous Effects of Invention

According to the present disclosure, deterioration of the device characteristics by exposure to high temperature can be suppressed.

### Brief Description of Drawings

[Fig. 1] Fig. 1 is a schematic cross-sectional view showing an example of a photoelectric conversion device according to an embodiment.
[Fig. 2] Fig. 2 is a schematic cross-sectional view showing another example of a photoelectric conversion device according to an embodiment.
[Fig. 3] Fig. 3 is an exemplary energy band diagram in the photoelectric conversion device shown in Fig. 2.
[Fig. 4] Fig. 4 is a diagram showing an example of a circuit configuration of an imaging apparatus according to an embodiment.
[Fig. 5] Fig. 5 is a schematic cross-sectional view showing an example of the device structure of a pixel in an imaging apparatus according to an embodiment.
[Fig. 6] Fig. 6 is a graph showing the measurement results of spectral sensitivity characteristics of photoelectric conversion devices in Example 1 and Comparative Example 1.

### Description of Embodiments

### (Underlying Knowledge Forming Basis of the Present Disclosure)

As described above, recently, photoelectric conversion devices and imaging apparatuses using organic semiconductor materials have been actively studied. In a photoelectric conversion device, when light enters a photoelectric conversion layer, pairs of electrons and holes, excitons, are generated by photoelectric conversion, the electrons and holes of the excitons are charge-separated, and the electron and the holes are collected by electrodes or the like.

A bulk heterojunction layer having a bulk heterojunction structure of a donor organic semiconductor and an acceptor organic semiconductor can realize a high photoelectric conversion efficiency and is therefore useful as a photoelectric conversion layer of a photoelectric conversion device and has been studied in recent years. It is known that in a bulk heterojunction layer, the excitons generated by absorption of light move in the donor organic semiconductor or the acceptor organic semiconductor in the exciton state and are charge-separated into electrons and holes at the interface between the donor organic semiconductor and the acceptor organic semiconductor. The exciton diffusion length through which excitons can diffuse varies depending on the material. The bulk heterojunction layer can realize a high photoelectric conversion efficiency by a domain structure in which domains of the donor organic semiconductor and the acceptor organic semiconductor where excitons are generated are smaller than the exciton diffusion length and the distance between the domains is close enough so that band conduction or hopping conduction of separated charges is possible.

However, the present inventors found that the following problems occur in a bulk heterojunction layer. In the bulk heterojunction layer, since the affinities between donor organic semiconductor molecules and between acceptor organic semiconductor molecules are high, aggregation and/or crystallization of donor organic semiconductor molecules and of acceptor organic semiconductor molecules is enhanced when heat is applied to the bulk heterojunction layer. As a result, the domain structure of the bulk heterojunction layer greatly changes from the structure immediately after the film formation. For example, the domains are enlarged to the exciton diffusion length or more by progress of aggregation and/or crystallization of donor organic semiconductor molecules and of acceptor organic semiconductor molecules, and a sufficient charge separation efficiency is not obtained. In addition, a trap level, which is a valley in the energy state, is generated between domains to decrease the efficiency of extracting charges separated. Consequently, in the photoelectric conversion device exposed to high temperature, the photoelectric conversion efficiency is decreased. Furthermore, aggregation and/or crystallization of donor organic semiconductor molecules and of acceptor organic semiconductor molecules causes roughness of the surface of the bulk heterojunction layer, which becomes a leakage source when an electric field is applied to the bulk heterojunction layer, and thereby dark current is likely to be generated in the photoelectric conversion device. Thus, in the bulk heterojunction layer exposed to high temperature, the photoelectric conversion efficiency and deterioration of the device characteristics such as dark current are likely to be caused by aggregation and so on of donor organic semiconductor molecules and of acceptor organic semiconductor molecules.

For example, NPL 2 discloses an organic thin film solar cell using an organic photoelectric conversion film in which [70]PCBM ([6,6]-phenyl-C71-butyric acid methyl ester) is mixed with [60]PCBM ([6,6]-phenyl-C61-butyric acid methyl ester) as an acceptor organic compound. However, it is difficult to sufficiently suppress degeneration of a photoelectric conversion layer by simply mixing fullerene derivatives having different numbers of carbon atoms, such as [60]PCBM and [70]PCBM.

From such knowledge, the present inventors focused on that in a photoelectric conversion device, aggregation and so on of donor organic semiconductor molecules and of acceptor organic semiconductor molecules can be suppressed by enhancing the affinity between the donor organic semiconductor and the acceptor organic semiconductor in a bulk heterojunction layer, and accomplished one aspect of the present disclosure.

Accordingly, the present disclosure provides a photoelectric conversion device or the like that can suppress deterioration of the device characteristics when exposed to high temperature, even when a bulk heterojunction layer is included.

### (Overview of one aspect according to the present disclosure)

The photoelectric conversion device according to one aspect of the present disclosure includes a first electrode, a second electrode facing the first electrode, and a photoelectric conversion layer located between the first electrode and the second electrode and including a bulk heterojunction layer containing a donor organic compound and an acceptor organic compound. The donor organic compound includes a first substituent. The acceptor organic compound includes an aromatic portion and a second substituent binding to the aromatic portion and having dipole-dipole interaction with the first substituent.

Consequently, dipole-dipole interaction is present between the first substituent and the second substituent, resulting in an increase in the affinity between the donor organic compound and the acceptor organic compound. Since the second substituent is bound to the aromatic portion, the aggregation force of the aromatic portion, which is particularly likely to aggregate, of the acceptor organic compound can be reduced. As a result, aggregation of the donor organic compound molecules and of the acceptor organic compound molecules is suppressed, the domain structure of the bulk heterojunction layer can be maintained. Accordingly, the photoelectric conversion device according to the present aspect can suppress deterioration of the device characteristics by exposure to high temperature.

For example, the first substituent and the second substituent may be each an alkoxy group, an alkylsulfanyl group, or a cyano group.

Consequently, dipole-dipole interaction between the first substituent and the second substituent is more likely to occur, and aggregation of the donor organic compound molecules and of the acceptor organic compound molecules can be effectively suppressed.

For example, the first substituent and the second substituent may be each an alkoxy group or a cyano group.

Consequently, dipole-dipole interaction between the first substituent and the second substituent is more likely to occur, and aggregation of the donor organic compound molecules and of the acceptor organic compound molecules can be more effectively suppressed.

For example, the donor organic compound may be a phthalocyanine derivative or a naphthalocyanine derivative.

Consequently, due to a wide π-conjugated system, aggregation of naphthalocyanine derivative molecules or phthalocyanine derivative molecules having high aggregative properties and crystallinity is suppressed, and the domain structure of the bulk heterojunction layer can be maintained.

For example, the donor organic compound may be a phthalocyanine derivative represented by the following formula (1) or a naphthalocyanine derivative represented by the following formula (2):

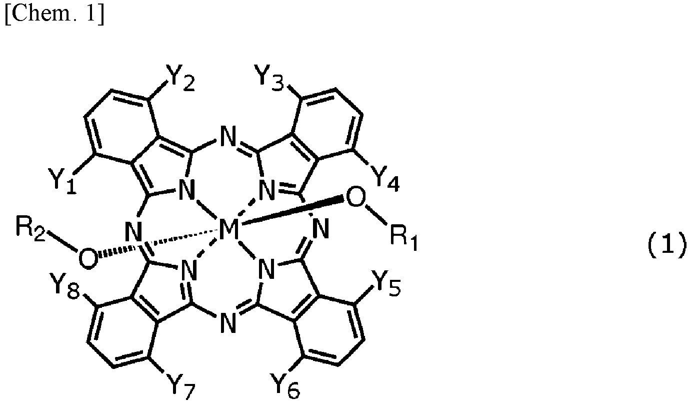

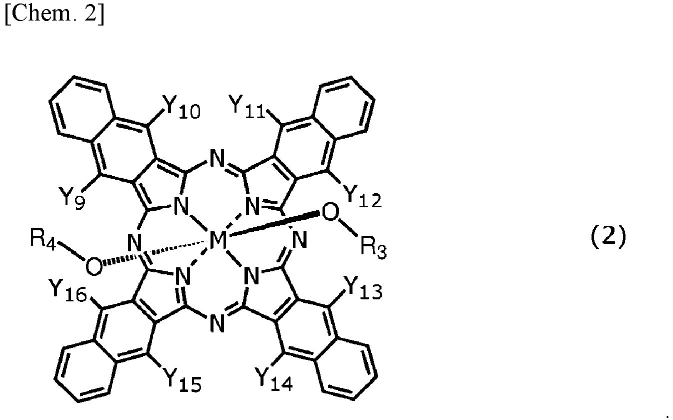

Here, Y₁ to Y₁₆ are each independently the first substituent, M is Si, Sn, or Ge, R₁ to R₄ are each independently any one of substituents represented by the following formulae (3) to (5), R₅ to R₇ are each independently an alkyl group or an aryl group, and R₈ to R₁₀ are each independently an aryl group. 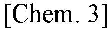 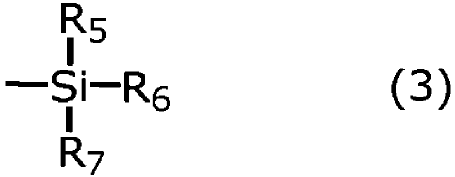 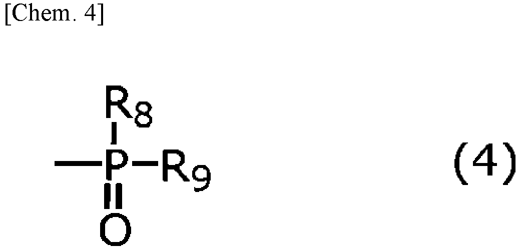 [Chem. 5]

-R₁₀ (5)

Consequently, since a plurality of first substituents binds to the aromatic portion of the phthalocyanine derivative or the phthalocyanine derivative, aggregation of naphthalocyanine derivative molecules or phthalocyanine derivative molecules is further likely to be suppressed.

For example, the acceptor organic compound may be a fullerene derivative.

Consequently, due to the fullerene portion, aggregation of the fullerene derivative having high aggregative properties is suppressed, and the domain structure of the bulk heterojunction layer can be maintained.

For example, the fullerene derivative may be C60 fullerene binding to the second substituent or [6,6]-phenyl-C61-butyric acid methyl ester ([60]PCBM) binding to the second substituent.

Consequently, the fullerene derivative can be easily synthesized.

For example, the photoelectric conversion device may further include a buffer layer located between the photoelectric conversion layer and at least one selected from the group consisting of the first electrode and the second electrode. The photoelectric conversion device may further include a first buffer layer located between the first electrode and the photoelectric conversion layer and a second buffer layer located between the second electrode and the photoelectric conversion layer.

Consequently, the buffer layer suppresses dark current due to injection of charges from an electrode or relieves damage and stress during formation of the electrode to improve the characteristics of the photoelectric conversion layer.

The imaging apparatus according to one aspect of the present disclosure includes a substrate and a pixel including a charge detection circuit provided in the substrate, a photoelectric conversion unit provided on the substrate, a charge storage node electrically connected to the charge detection circuit and the photoelectric conversion unit. The photoelectric conversion unit includes the above-described photoelectric conversion device.

Consequently, since the imaging apparatus includes the photoelectric conversion device, deterioration of the device characteristics by heating can be suppressed.

Embodiments will now be specifically described with reference to the drawings.

It should be noted that the embodiments described below are all comprehensive or specific examples. The numerical values, shapes, materials, components, arrangement positions and connection configurations of components, steps, order of steps, and so on shown in the following embodiments are examples and are not intended to limit the present disclosure. In addition, among the components in the following embodiments, the components that are not mentioned in independent claims will be described as optional components. Each drawing is not necessarily strictly illustrated. Accordingly, for example, in each drawing, the scale or the like is not necessarily identical. In addition, in each drawing, substantially the same configurations are given with the same reference signs, and overlapping explanations are omitted or simplified.

In the present specification, terms indicating relationships between elements, terms indicating shapes of elements, and numerical ranges are not expressions that only have strict meanings and are expressions that include substantially the same range, for example, a difference of about several percent.

In addition, in the present specification, the terms "upper" and "lower" do not refer to the upward (vertically upward) and the downward (vertically downward) in absolute spatial perception and are used as terms that are defined by relative positional relationship based on the stacking order in a stacking structure. Specifically, the light receiving side of an imaging apparatus is referred to as "upper", and the side opposite to the light receiving side is referred to as "lower". The terms such as "upper" and "lower" are used only to specify the mutual arrangement of members and are not intended to limit the posture of an imaging apparatus when it is used. In addition, the terms "upper" and "lower" are applied not only to when two components are arranged with a gap therebetween and another component is present between the two components but also to when two components are arranged to adhere to each other and are in contact with each other.

### (Embodiments)

### [Photoelectric conversion device]

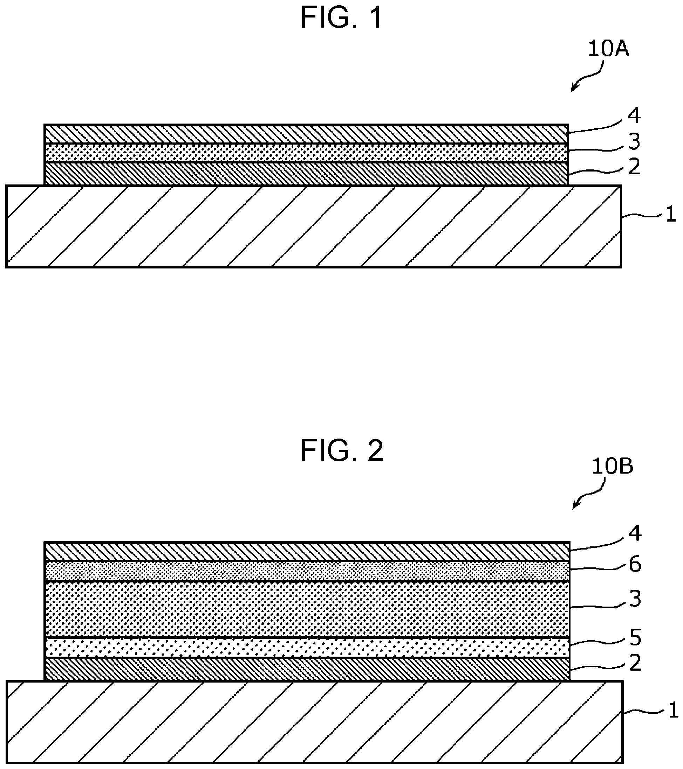

A photoelectric conversion device according to the present embodiment will now be described with reference to the drawings. The photoelectric conversion device according to the present embodiment is, for example, a photoelectric conversion device of a charge-readout system. Fig. 1 is a schematic cross-sectional view showing a photoelectric conversion device 10A as an example of the photoelectric conversion device according to the present embodiment.

The photoelectric conversion device 10A according to the present embodiment includes a pair of electrodes, an upper electrode 4 and a lower electrode 2, disposed so as to face each other and a photoelectric conversion layer 3 located between the pair of electrodes. The photoelectric conversion layer 3 is constituted of a bulk heterojunction layer containing a donor organic compound and an acceptor organic compound. The bulk heterojunction layer is a layer having a bulk heterojunction structure of a donor organic semiconductor containing the donor organic compound and an acceptor organic semiconductor containing the acceptor organic compound, for example, the whole layer is a bulk heterojunction structure.

The photoelectric conversion device 10A according to the present embodiment is, for example, supported by a supporting substrate 1. The supporting substrate 1 is transparent to, for example, light of a wavelength that can be absorbed by the photoelectric conversion layer 3, and light enters the photoelectric conversion device 10A through the supporting substrate 1. The supporting substrate 1 may be a substrate that is used in general photoelectric conversion devices and may be, for example, a glass substrate, a quartz substrate, a silicon substrate, a semiconductor substrate, or a plastic substrate. In the present specification, the term "transparent" means transmitting light of a wavelength that can be absorbed by the photoelectric conversion layer 3 at least partially, and it is not essential to transmit light over the entire wavelength range.

Each component of the photoelectric conversion device 10A according to the present embodiment will now be described.

First, the photoelectric conversion layer 3 will be described. The photoelectric conversion layer 3 absorbs light entered the photoelectric conversion device 10A and generates a pair of charges (exciton) by photoelectric conversion. The generated pair of charges are separated and captured by the upper electrode 4 and the lower electrode 2. The photoelectric conversion layer 3 is constituted of a bulk heterojunction layer in which a donor organic semiconductor containing a donor organic compound and an acceptor organic semiconductor containing an acceptor organic compound are mixed. In the bulk heterojunction layer, a pair of charges may be generated in one of the donor organic semiconductor and the acceptor organic semiconductor or may be generated in both the donor organic semiconductor and the acceptor organic semiconductor.

The donor organic semiconductor and acceptor organic semiconductor according to the present embodiment will now be specifically described.

The donor organic semiconductor is mainly constituted of a donor organic compound that is represented by a hole-transporting organic compound and has a property of easily donating electrons. In more details, the donor organic compound is an organic compound having a smaller ionization potential when two organic materials are used in contact with each other. The donor organic compound includes a first substituent having dipole-dipole interaction with a second substituent described later. The donor organic compound includes, for example, an aromatic portion. The first substituent is, for example, a substituent that binds to the aromatic portion. That is, the first substituent is a substituent of the aromatic portion of the donor organic compound.

As the donor organic compound, for example, a triarylamine compound, a benzidine compound, a pyrazoline compound, a styrylamine compound, a hydrazone compound, a triphenylmethane compound, a carbazole compound, a polysilane compound, a thiophene compound, a phthalocyanine compound, a naphthalocyanine compound, a cyanine compound, a merocyanine compound, an oxonol compound, a polyamine compound, an indole compound, a pyrrole compound, a pyrazole compound, a polyarylene compound, a condensed aromatic carbocyclic compound (a naphthalene derivative, an anthracene derivative, a phenanthrene derivative, a tetracene derivative, a pyrene derivative, a perylene derivative, or a fluoranthene derivative), and a metal complex whose ligand is a nitrogen-containing heterocyclic compound can be used.

The acceptor organic semiconductor is mainly constituted of an acceptor organic compound that is represented by an electron-transporting organic compound and has a property of easily accepting electrons. In more details, the acceptor organic compound is an organic compound having a higher electron affinity when two organic compounds are used in contact with each other. The acceptor organic compound includes a second substituent having dipole-dipole interaction with the first substituent described above. The acceptor organic compound includes, for example, an aromatic portion. The second substituent is, for example, a substituent that binds to the aromatic portion. That is, the second substituent is a substituent of the aromatic portion of the acceptor organic compound.

As the acceptor organic compound, for example, a fullerene derivative, a condensed aromatic carbocyclic compound (e.g., a naphthalene derivative, an anthracene derivative, a phenanthrene derivative, a tetracene derivative, a pyrene derivative, a perylene derivative, and a fluoranthene derivative), 5- to 7-membered heterocyclic compounds containing a nitrogen atom, an oxygen atom, or a sulfur atom (e.g., pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, oxazole, indazole, benzimidazole, benzotriazole, benzoxazole, benzothiazole, carbazole, purine, triazolopyridazine, triazolopyrimidine, tetrazaindene, oxadiazole, imidazopyridine, pyrrolidine, pyrrolopyridine, thiadiazolopyridine, dibenzazepine, and tribenzazepine), a polyarylene compound, a fluorene compound, a cyclopentadiene compound, a silyl compound, and a metal complex whose ligand is a nitrogen-containing heterocyclic compound can be used.

Here, the first substituent and the second substituent will be described. As described above, dipole-dipole interaction is present between the first substituent and the second substituent. Consequently, the affinity between the donor organic compound and the acceptor organic compound is increased, and even when the photoelectric conversion layer 3 is exposed to high temperature, aggregation and so on of donor organic compound molecules and of acceptor organic compound molecules are suppressed.

As described above, the first substituent and the second substituent each bind to, for example, the aromatic portion. Consequently, since the first substituent and the second substituent bind to the aromatic portion, which is particularly likely to aggregate by π-π interaction, even when the photoelectric conversion layer 3 is exposed to high temperature, aggregation and so on of donor organic compound molecules and of acceptor organic compound molecules are effectively suppressed.

The first substituent and the second substituent are each, for example, a substituent containing at least one atom selected from the group consisting of an oxygen atom (O), a nitrogen atom (N), a sulfur atom (S), a selenium atom (Se), a boron atom (B), a phosphorus atom (P), a fluorine atom (F), a chlorine atom (Cl), a bromine atom (Br), and an iodine atom (I). Examples of each of the first substituent and the second substituent include an alkoxy group, an alkylsulfanyl group, a cyano group, a nitro group, a halogen group, an amino group, an alkylamino group, a dialkylamino group, a carbonyl group, and a hydroxyl group. The first substituent and the second substituent may be each an alkyl group in which at least one hydrogen atom is substituted by a halogen atom, such as a trifluoromethyl group.

From the viewpoint of ease of synthesis and ease of occurrence of dipole-dipole interaction, the first substituent and the second substituent may be each an alkoxy group, an alkylsulfanyl group, or a cyano group, in particular, an alkoxy group or a cyano group.

The first substituent and the second substituent may be each an acyclic substituent or a substituent having a cyclic skeleton containing at least one selected from the group consisting of oxygen (O), nitrogen (N), sulfur (S), selenium (Se), boron (B), and phosphorus (P). The first substituent and the second substituent each include, as a cyclic skeleton, for example, pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, oxazole, indazole, benzimidazole, benzotriazole, benzoxazole, benzothiazole, carbazole, purine, triazolopyridazine, triazolopyrimidine, tetrazaindene, oxadiazole, imidazopyridine, pyrrolidine, pyrrolopyridine, thiadiazolopyridine, dibenzazepine, or tribenzazepine.

The first substituent and the second substituent may be, for example, substituents having the same structural formula, substituents in the same classification as mentioned in the specific examples above, substituents having different structural formulae, or substituents in different classifications. From the viewpoint of ease of occurrence of dipole-dipole interaction, the first substituent and the second substituent may be substituents in the same classification.

The donor organic compound may include a plurality of first substituents. In such a case, the plurality of first substituents may all have the same structural formula or may all be in the same classification, or at least one of the structural formulae may be different from the others or may be in a different classification. From the viewpoint of ease of synthesis, the plurality of first substituents may all have the same structural formula.

The acceptor organic compound may include a plurality of second substituent. In such a case, the plurality of second substituents may all have the same structural formula or may all be in the same classification, or at least one of the structural formulae may be different from the others or may be in a different classification. From the viewpoint of ease of synthesis, the plurality of second substituents may all have the same structural formula.

Next, specific examples of the donor organic compound and acceptor organic compound will be described.

In the present embodiment, the donor organic compound is, for example, a phthalocyanine derivative including a first substituent or a naphthalocyanine derivative including a first substituent. Consequently, since the donor organic compound has a wide π-conjugated system, the photoelectric conversion efficiency of the photoelectric conversion device can be enhanced, and also aggregation of the donor organic compound by heating can be suppressed even when a phthalocyanine derivative or naphthalocyanine derivative, which are likely to aggregate, is used as the donor organic compound. In both the phthalocyanine derivative and the naphthalocyanine derivative, the first substituent binds to, for example, the aromatic portion. Alternatively, the phthalocyanine derivative and the naphthalocyanine derivative each have the first substituent, for example, at the α-position side chain. Alternatively, the phthalocyanine derivative and the naphthalocyanine derivative may each have the first substituent at a part of the axial ligand coordinated to the central metal.

The phthalocyanine derivative and the naphthalocyanine derivative may include a plurality of first substituents. In the present embodiment, for example, the donor organic semiconductor includes a phthalocyanine derivative or a naphthalocyanine derivative as the main component.

In the present embodiment, the donor organic compound is, for example, a phthalocyanine derivative represented by the following formula (1) or a naphthalocyanine derivative represented by the following formula (2):

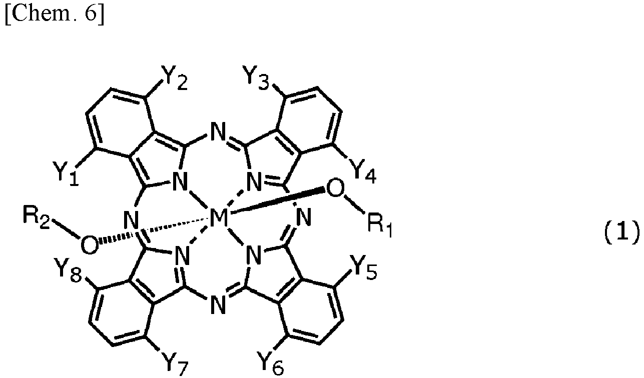

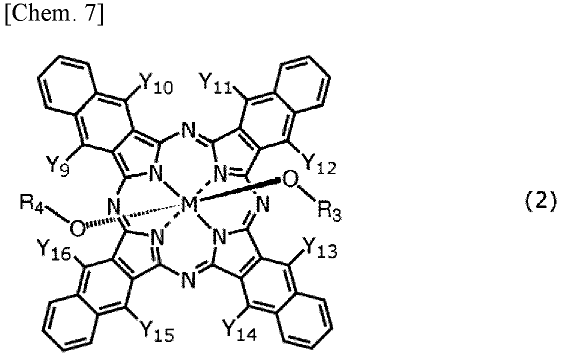

Here, Y₁ to Y₁₆ are each independently a first substituent, M is Si, Sn, or Ge, R₁ to R₄ are each independently any one of substituents represented by the following formulae (3) to (5). Y₁ to Y₈ and Y₉ to Y₁₆ may be first substituents all having the same structural formula or first substituents all belonging to the same classification or may include a first substituent having a structural formula different from those of the others or a first substituent belonging to a classification different from those of the others. From the viewpoint of ease of synthesis, Y₁ to Y₈ and Y₉ to Y₁₆ may be first substituents all having the same structural formula. 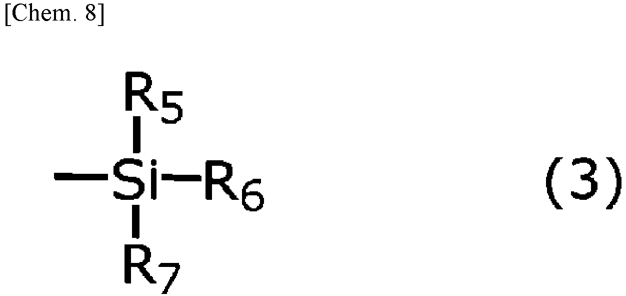 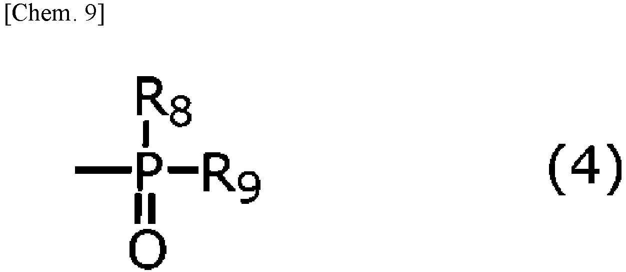 [Chem. 10]

-R₁₀ (5)

R₅ to R₇ are each independently an alkyl group or an aryl group, and R₈ to R₁₀ are each independently an aryl group. In each of at least one selected from the group consisting of R₅ to R₇ in the formula (3), at least one selected from the group consisting of R₈ and R₉ in the formula (4), and R₁₀ in the formula (5), at least one hydrogen atom may be substituted by a first substituent. In this case, Y₁ to Y₈ and Y₉ to Y₁₆ may be each hydrogen or a substituent other than the first substituent, such as a hydrocarbon group.

Thus, the aggregation force of π-conjugated structure of a phthalocyanine derivative or naphthalocyanine derivative, which is easily aggregate, can be suppressed by introducing the first substituent into the α-position side chain, Y₁ to Y₁₆.

In the formulae (1) and formula (2), M may be Si, and the first substituents, Y₁ to Y₁₆, may be each an alkoxy group or an alkylsulfanyl group. In the formula (1), the first substituents, Y₁ to Y₈, may be each an alkylsulfanyl group. In the formula (2), the first substituents, Y₉ to Y₁₆, may be each an alkoxy group. Consequently, the phthalocyanine derivative or the naphthalocyanine derivative can be easily synthesized. Furthermore, since the phthalocyanine derivative and the naphthalocyanine derivative each include an electron-donating alkoxy group or an alkylsulfanyl group in the α-position side chain, the absorbance wavelength shifts to longer wavelength, and the absorbance coefficient is likely to become high in the near-infrared light region.

In each of at least one selected from the group consisting of R₅ to R₇ in the formula (3), at least one selected from the group consisting of R₈ and R₉ in the formula (4), and R₁₀ in the formula (5), at least one hydrogen atom may be substituted by an electron-withdrawing group. Consequently, the electron-drawing property of the axial ligand present in the phthalocyanine derivative or naphthalocyanine derivative is enhanced, the electron density of the phthalocyanine ring or naphthalocyanine ring is decreased, and the energy band gap of the phthalocyanine derivative or naphthalocyanine derivative is narrowed. As a result, the absorbance wavelength of the phthalocyanine derivative or naphthalocyanine derivative further shifts to longer wavelength, and also dark current in the imaging apparatus can be suppressed. Examples of the electron-withdrawing group include a cyano group, a fluoro group, and a carbonyl group. The electron-withdrawing group may be a cyano group or a fluoro group from the viewpoint of the height of the electron-withdrawing property.

In the present embodiment, the phthalocyanine derivative or naphthalocyanine derivative may be, for example, any one of the compounds represented by the following structural formulae (6) to (15):

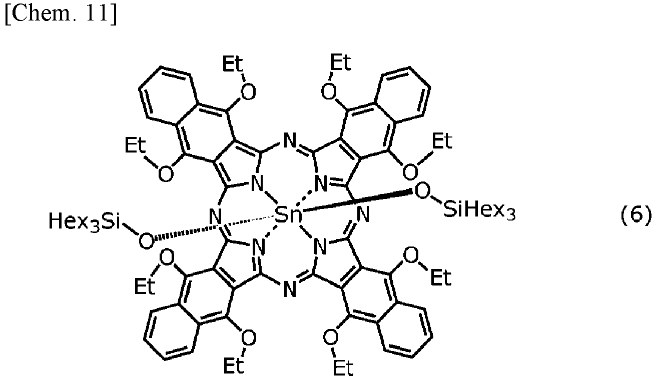

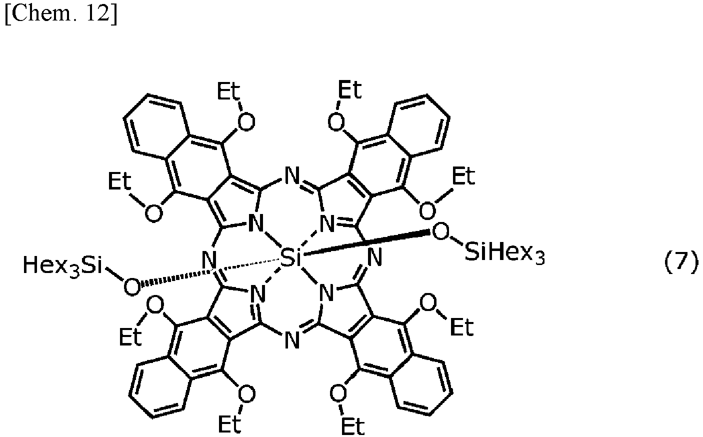

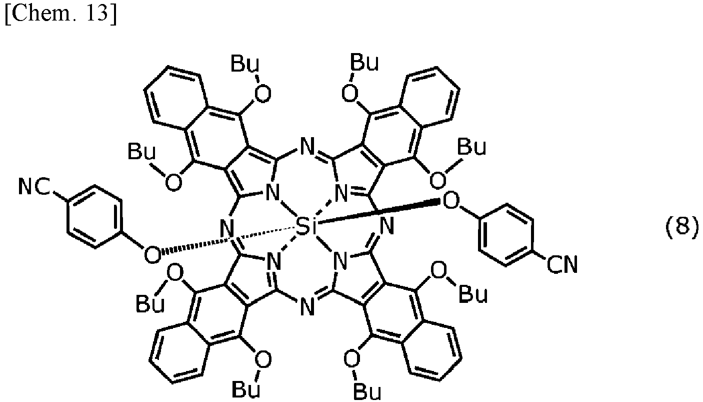

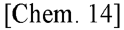

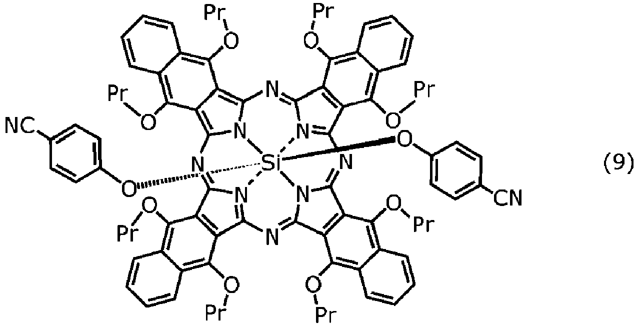

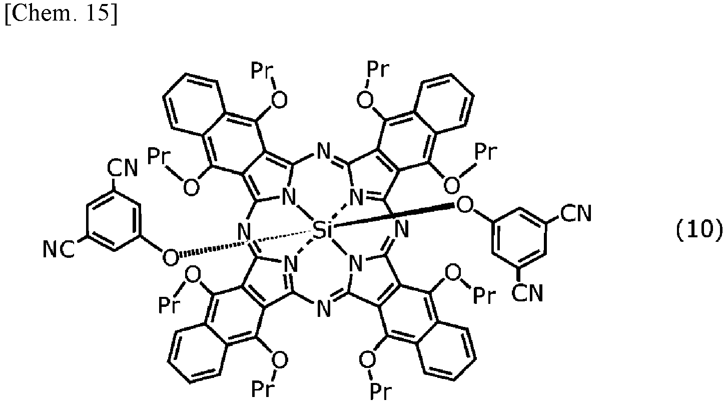

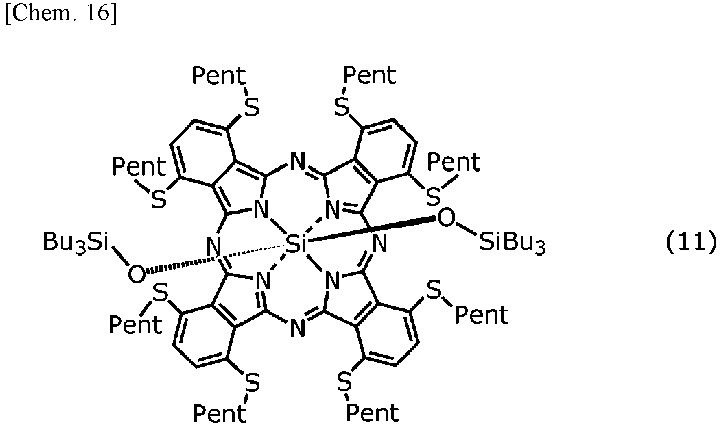

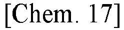

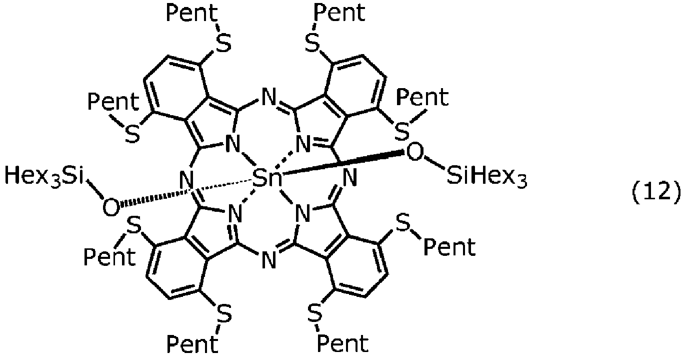

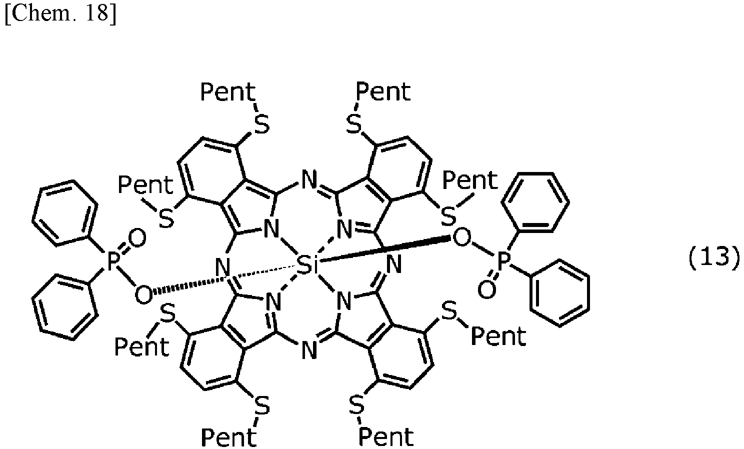

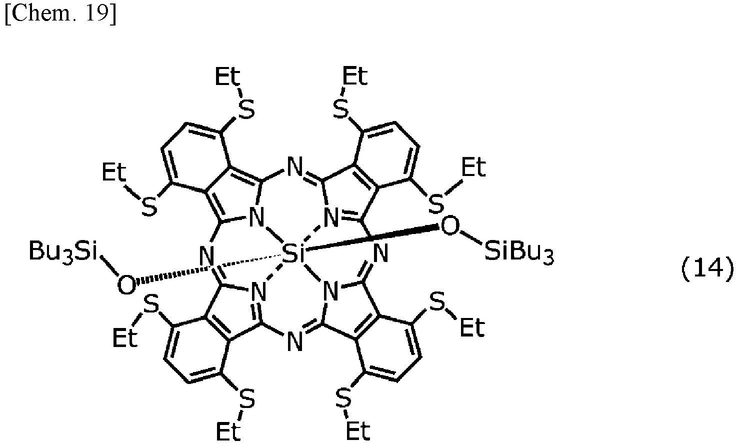

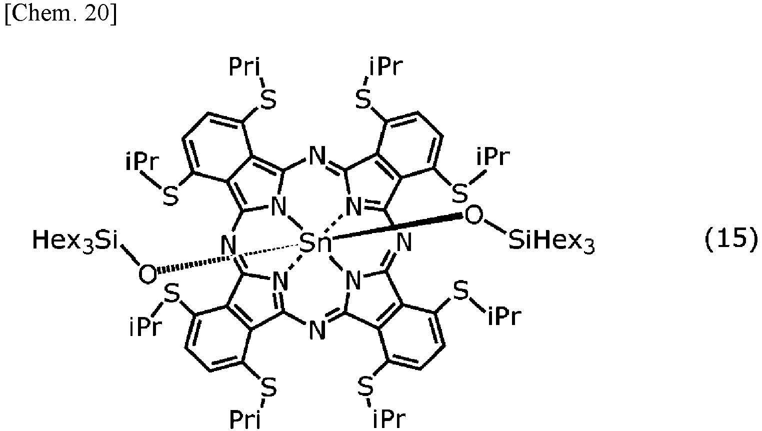

When the phthalocyanine derivative or naphthalocyanine derivative is any one of the compounds represented by the structural formulae (6) to (15) above, a high photoelectric conversion efficiency can be obtained in the near-infrared light region. The compounds represented by the structural formulae (6) to (15) above can be synthesized by known synthetic methods or the synthetic methods shown in the following Examples.

The donor organic semiconductor may include materials other than the phthalocyanine derivative and naphthalocyanine derivative. For example, the donor organic semiconductor may include at least one of the organic compounds exemplified as the donor organic compounds above.

In the present embodiment, the acceptor organic compound is, for example, a fullerene derivative having a second substituent. In the present embodiment, for example, the acceptor organic semiconductor contains a fullerene derivative as the main component. Consequently, even when a fullerene derivative having a fullerene portion which is likely to aggregate is used as the acceptor organic compound, aggregation of the acceptor organic compound by heating can be suppressed. The fullerene derivative may include a plurality of second substituents. In the fullerene derivative, the second substituent binds to, for example, an aromatic portion. In the present embodiment, for example, the acceptor organic semiconductor contains the fullerene derivative as the main component.

From the viewpoint of ease of synthesis, in the fullerene derivative, the second substituent may be an alkoxy group.

Examples of the fullerene derivative include fullerenes such as C60 fullerene and C70 fullerene having the second substituent attached thereto. In such a case, the second substituent binds to the fullerene portion. The fullerenes in which the second substituent is bound to the fullerene portion can be synthesized by, for example, addition reaction of the second substituent to fullerene such as the method described in NPL 3.

Examples of the fullerene derivative include PCBMs such as [60] PCBM and [70] PCBM having the second substituent attached thereto. The PCBMs are compounds in which phenylbutyric acid methyl esters are added to fullerene portions with various numbers of carbon atoms through a 3-membered ring structure. In such cases, the second substituent binds to the phenyl group of the phenylbutyric acid methyl ester portion or the fullerene portion. From the viewpoint of ease of synthesis, the second substituent may bind to the phenyl group of the phenylbutyric acid methyl ester portion. When the fullerene derivative includes a plurality of second substituents, the plurality of second substituents each binds to one of the phenyl group of the phenylbutyric acid methyl ester portion or the fullerene portion. In the PCBMs, a plurality of phenylbutyric acid methyl esters may be added to the fullerene portion through a 3-membered ring structure.

The PCBMs in which a second substituent is bound to the phenyl group of the phenylbutyric acid methyl ester portion can be synthesized by, for example, a nucleophilic addition reaction of a phenylbutyric acid methyl ester derivative in which the second substituent is bound to the phenyl group in advance to fullerenes.

The fullerene derivative is C60 fullerene having the second substituent bound thereto or [60] PCBM having the second substituent bound thereto. Consequently, the fullerene derivative can be easily synthesized.

In the present embodiment, the fullerene derivative may be, for example, any one of the compounds represented by the following structural formulae (16) to (19). The compounds represented by the following structural formulae (16) to (19) are each [60] PCBM having the second substituent bound thereto. In the present specification, in the fullerene portion in the structural formula, such as the fullerene derivative, not all carbon atoms are drawn, and some of carbon atoms are omitted, for the convenience of illustrating on a plane.

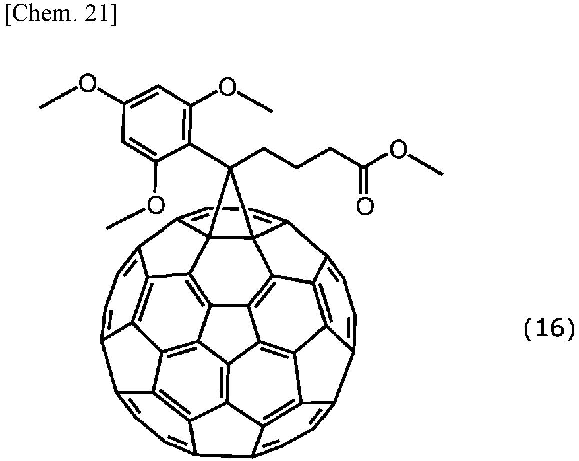

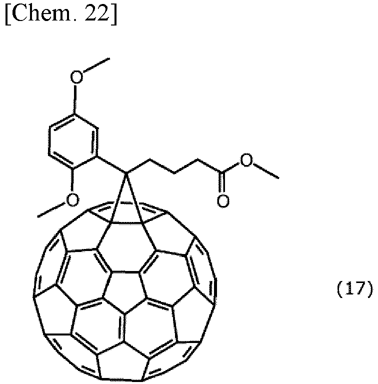

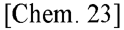

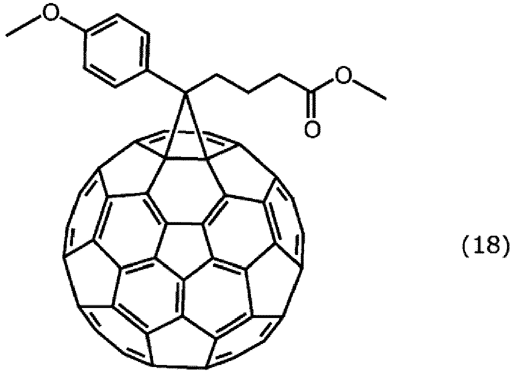

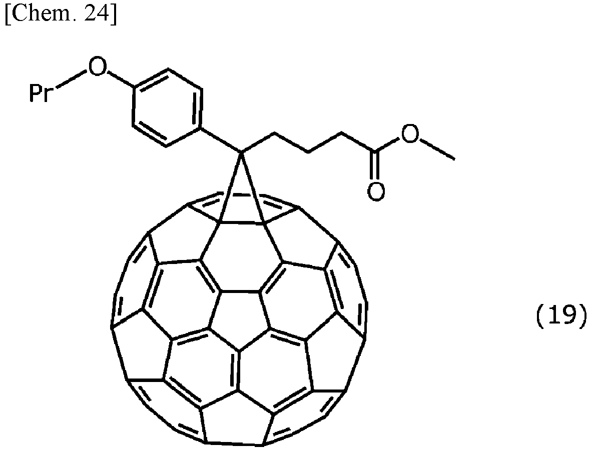

The acceptor organic semiconductor may include a material other than fullerene derivatives. For example, the acceptor organic semiconductor may include at least one of the organic compounds exemplified as the acceptor organic compound above.

As the method for producing the photoelectric conversion layer 3, for example, a coating method such as spin coating or a vacuum evaporation method in which a material for a film is evaporated by heating under vacuum to deposit it on a substrate can be used. When considering prevention of contamination of impurities and multi-layering for high functionality with a higher degree of freedom, an evaporation may be used. The evaporation apparatus to be used may be a commercially available apparatus. The temperature of the evaporation source during evaporation may be 100°C or more and 500°C or less or 150°C or more and 400°C or less. The degree of vacuum during evaporation may be 1 × 10⁻⁴ Pa or more and 1 Pa or less or 1 × 10⁻³ Pa or more and 0.1 Pa or less. A method for increasing the evaporation speed by adding metal microparticles or the like to the evaporation source may be used.

The blending ratio of the materials for the photoelectric conversion layer 3 is shown by a weight ratio in the coating method and is shown by a volume ratio in the evaporation method. More specifically, in the coating method, the blending ratio is prescribed by the weight of each material when a solution is prepared. In the evaporation method, the blending ratio of each material is prescribed while monitoring the deposition film thickness of each material with a film thickness meter during the evaporation.

The upper electrode 4 and the lower electrode 2 will be then described.

At least one of the upper electrode 4 and the lower electrode 2 is a transparent electrode made of a conducting material transparent to light with a response wavelength. Only the upper electrode 4 may be a transparent electrode, or only the lower electrode 2 may be a transparent electrode, or both the upper electrode 4 and the lower electrode 2 may be transparent electrodes. A bias voltage is applied to the lower electrode 2 and the upper electrode 4 by wiring (not shown). For example, the polarity of the bias voltage is determined such that among the charges generated in the photoelectric conversion layer 3, electrons move to the upper electrode 4 and holes move to the lower electrode 2. The bias voltage may be set such that among the charges generated in the photoelectric conversion layer 3, holes move to the upper electrode 4 and electrons move to the lower electrode 2.

The bias voltage may be applied such that the electric field occurring in the photoelectric conversion device 10A, i.e., the strength value obtained by dividing the voltage value to be applied by the distance between the lower electrode 2 and the upper electrode 4, is within a range of 1.0 × 10³ V/cm or more and 1.0 × 10⁷ V/cm or less or within a range of 1.0 × 10⁴ V/cm or more and 1.0 × 10⁶ V/cm or less. By thus adjusting the level of the bias voltage, it is possible to efficiently move the charges to the upper electrode 4 and to extract the signals according to the charges to the outside.

As the materials for the lower electrode 2 and the upper electrode 4, transparent conducting oxides (TCOs) having a high transmittance of light in the near-infrared light region and a small resistance value may be used. A metal thin film of Au or the like can also be used as the transparent electrode, but in order to obtain a light transmittance of 90% or more in the near-infrared light region, the resistance value may significantly increase compared to when a transparent electrode is produced so as to have a transmittance of 60% to 80%. Accordingly, a transparent electrode having high transparency to near-infrared light and a small resistance value can be obtained by using a TCO rather than by using a metal material such as Au. The TCO is not particularly limited, and examples thereof include indium tin oxide (ITO), indium zinc oxide (IZO), aluminum-doped zinc oxide (AZO), fluorine-doped tin oxide (FTO), SnO₂, TiO₂, and ZnO₂. The lower electrode 2 and the upper electrode 4 may be produced by using metal materials, such as TCO and Au, alone or in combination of two or more thereof, appropriately, according to a desired transmittance.

The materials for the lower electrode 2 and the upper electrode 4 are not limited to the above-mentioned conducting materials transparent to near-infrared light, and other materials may be used.

The lower electrode 2 and the upper electrode 4 are produced by various methods depending on the materials to be used. For example, when ITO is used, a chemical reaction method such as an electron beam method, a sputtering method, a resistance heating evaporation method, or a sol-gel method or a method such as application of a dispersion of indium tin oxide may be used. In such a case, after formation of an ITO film, UV-ozone treatment, plasma treatment, or the like may be further performed.

According to the photoelectric conversion device 10A, for example, photoelectric conversion is caused in the photoelectric conversion layer 3 by the light incident through the supporting substrate 1 and the lower electrode 2 and/or the light incident through the upper electrode 4. In the consequently generated pairs of charges, i.e., pairs of electrons and holes, holes are collected by the lower electrode 2, and electrons are collected by the upper electrode 4. Accordingly, for example, the light incident on the photoelectric conversion device 10A can be detected by measuring the potential of the lower electrode 2.

The photoelectric conversion device 10A may further include a buffer layer 5 (see Fig. 2) and a buffer layer 6 (see Fig. 2) described later. By sandwiching the photoelectric conversion layer 3 by the buffer layer 5 and the buffer layer 6, for example, the injection of electrons from the lower electrode 2 into the photoelectric conversion layer 3 can be prevented, and the injection of holes from the upper electrode 4 into the photoelectric conversion layer 3 can be prevented. Consequently, dark current can be suppressed. The details of the buffer layer 5 and the buffer layer 6 will be described later, and descriptions thereof are omitted here.

As described above, the photoelectric conversion device 10A according to the present embodiment includes a photoelectric conversion layer 3 including a bulk heterojunction layer containing a donor organic compound having a first substituent and an acceptor organic compound in which a second substituent having dipole-dipole interaction with the first substituent is bound to the aromatic portion. Since the sizes of domains and the distances between domains in the donor organic compound and the acceptor organic compound contained in the bulk heterojunction layer are appropriate, as described above, high device characteristics can be expressed. That is, since the donor organic compound and the acceptor organic compound are appropriately dispersed in the bulk heterojunction layer, high device characteristics can be expressed. In general, since the affinities between donor organic compound molecules and between acceptor organic compound molecules are high, aggregation and so on occur when the compounds become easier to move due to heat or the like. In the photoelectric conversion device 10A, since there is dipole-dipole interaction between the first substituent and the second substituent, the affinity between the donor organic compound and the acceptor organic compound is enhanced. Accordingly, even when the photoelectric conversion device 10A is exposed to high temperature, aggregation of the donor organic compound and of the acceptor organic compound is suppressed, and a state in which the donor organic compound and the acceptor organic compound are appropriately dispersed is maintained. Accordingly, the photoelectric conversion device 10A can suppress deterioration of the device characteristics caused by aggregation of the donor organic compound molecules and of the acceptor organic compound molecules.

Subsequently, another example of the photoelectric conversion device according to the present embodiment will be described. Fig. 2 is a schematic cross-sectional view showing a photoelectric conversion device 10B as another example of the photoelectric conversion device according to the present embodiment.

In the photoelectric conversion device 10B shown in Fig. 2, the components that are the same as those of the photoelectric conversion device 10A shown in Fig. 1 are denoted by the same reference signs.

As shown in Fig. 2, the photoelectric conversion device 10B according to the present embodiment includes a lower electrode 2 and an upper electrode 4 as a pair of electrodes and a photoelectric conversion layer 3 provided between the pair of electrodes. The photoelectric conversion device 10B further includes a buffer layer 5 disposed between the lower electrode 2 and the photoelectric conversion layer 3 and a buffer layer 6 disposed between the upper electrode 4 and the photoelectric conversion layer 3. The details of the lower electrode 2, the upper electrode 4, and the photoelectric conversion layer 3 are as described in the explanation of the photoelectric conversion device 10A, and therefore the description thereof is omitted here.

The buffer layer 5 is provided, for example, for reducing the dark current due to injection of electrons from the lower electrode 2 and suppresses the injection of electrons from the lower electrode 2 into the photoelectric conversion layer 3. That is, the buffer layer 5 may be an electron blocking layer. The buffer layer 5 has also a function of transporting the holes generated in the photoelectric conversion layer 3 to the lower electrode 2. As the buffer layer 5, a donor semiconductor or a hole-transporting organic compound, such as the materials mentioned in the above-described donor organic semiconductor, can be used. The buffer layer 5 may be a protective layer for protecting the photoelectric conversion layer 3 from the stress, chemical materials, heat, and so on when electrodes and so on are formed.

The buffer layer 6 is provided, for example, for reducing the dark current due to injection of holes from the upper electrode 4 and suppresses the injection of holes from the upper electrode 4 into the photoelectric conversion layer 3. That is, the buffer layer 6 may be a hole blocking layer. The buffer layer 6 has also a function of transporting the electrons generated in the photoelectric conversion layer 3 to the upper electrode 4. As the material for the buffer layer 6, for example, an organic substance such as copper phthalocyanine, ClAlPc (chloroaluminum phthalocyanine), PTCDA (3,4,9,10-perylenetetracarboxylic dianhydride), an acetylacetonate ligand, BCP (bathocuproine), and Alq (tris(8-quinolinolate)aluminum); or an organometallic compound; or an inorganic substance such as MgAg and MgO; can be used. As the buffer layer 6, an acceptor semiconductor or an electron-transporting organic compound, such as materials mentioned in the above-described acceptor organic semiconductor, can also be used. The buffer layer 6 may be a protective layer for protecting the photoelectric conversion layer 3 from the stress, chemical materials, heat, and so on when electrodes and so on are formed.

In the buffer layer 6, in order not to prevent the light absorption of the photoelectric conversion layer 3, the transmittance of light in the wavelength region for photoelectric conversion may be high, a material not having absorption in the visible light region may be selected, or the thickness of the buffer layer 6 may be reduced. The thickness of the buffer layer 6 depends on the configuration of the photoelectric conversion layer 3, the thickness of the upper electrode 4, and so on, but may be, for example, 2 nm or more and 50 nm or less.

When the buffer layer 5 is provided, the material for the lower electrode 2 is selected from the above-mentioned materials considering the adhesion with the buffer layer 5, electron affinity, ionization potential, stability, and so on. The same also applies to the upper electrode 4 when the buffer layer 6 is provided.

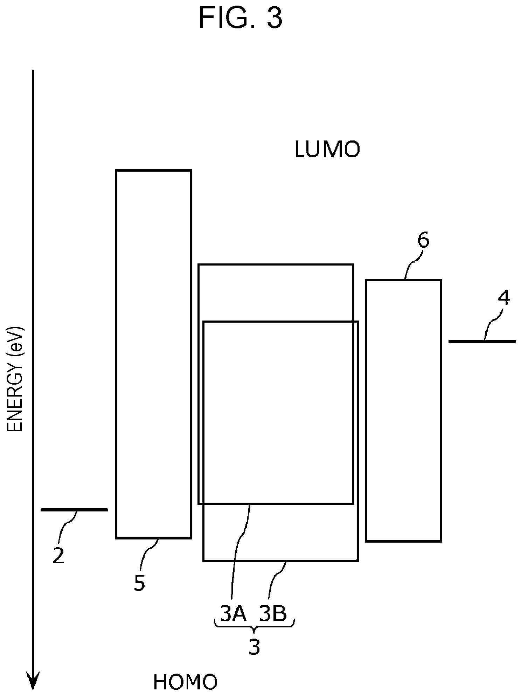

Fig. 3 shows an example of a schematic energy band diagram of the photoelectric conversion device 10B having the configuration shown in Fig. 2.

As shown in Fig. 3, in the photoelectric conversion device 10B, for example, the highest occupied molecular orbital (HOMO) energy level of the buffer layer 5 is lower than the HOMO energy level of the donor organic semiconductor 3A contained in the photoelectric conversion layer 3. In addition, for example, the lowest unoccupied molecular orbital (LUMO) energy level of the buffer layer 5 is higher than the LUMO energy level of the donor organic semiconductor 3A.

In the photoelectric conversion device 10B, for example, the LUMO level of the buffer layer 6 is higher than the LUMO energy level of the acceptor organic semiconductor 3B contained in the photoelectric conversion layer 3.

In the photoelectric conversion device 10B, the positions of the buffer layer 5 and the buffer layer 6 may be interchanged. That is, the buffer layer 5 may be disposed between the upper electrode 4 and the photoelectric conversion layer 3, and the buffer layer 6 may be disposed between the lower electrode 2 and the photoelectric conversion layer 3. The photoelectric conversion device 10B may be provided with either one of the buffer layer 5 and the buffer layer 6.

### [Imaging apparatus]

Then, an imaging apparatus according to the present embodiment will be described with reference to the drawings. The imaging apparatus according to the present embodiment is, for example, an imaging apparatus of a charge-readout system.

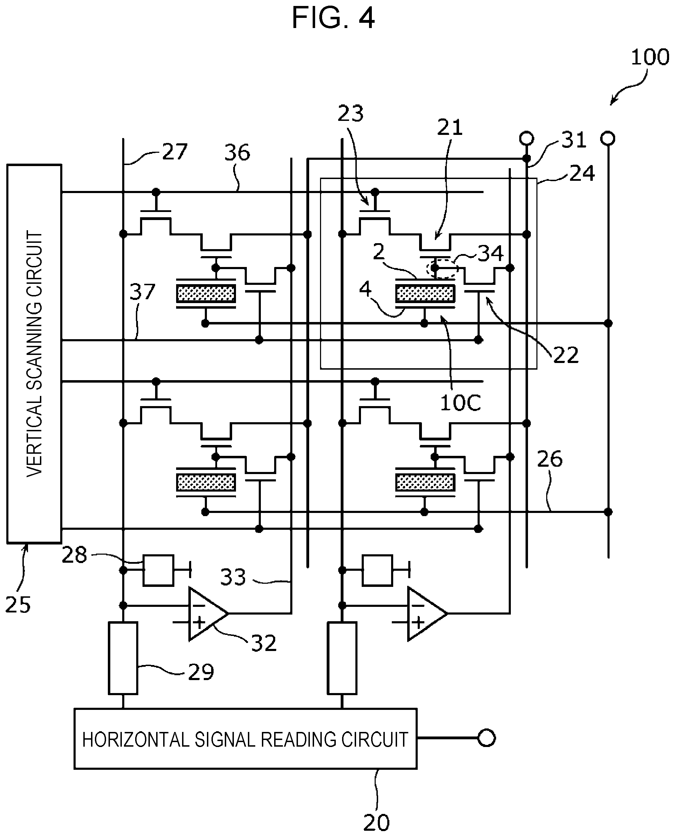

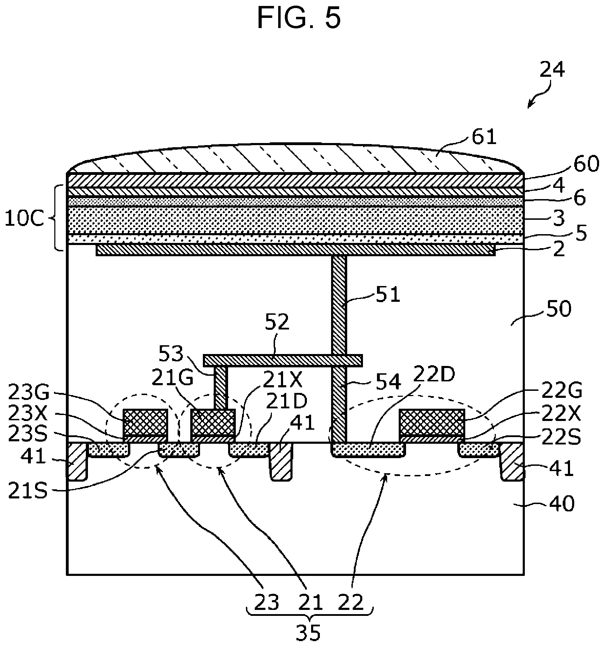

The imaging apparatus according to the present embodiment will be described using Figs. 4 and 5. Fig. 4 is a diagram showing an example of a circuit configuration of an imaging apparatus 100 according to the present embodiment. Fig. 5 is a schematic cross-sectional view showing an example of the device structure of a pixel 24 in the imaging apparatus 100 according to the present embodiment.

The imaging apparatus 100 according to the present embodiment includes a semiconductor substrate 40 as an example of the substrate and a pixel 24 including a charge detection circuit 35 provided to the semiconductor substrate 40, a photoelectric conversion unit 10C provided on the semiconductor substrate 40, and a charge storage node 34 electrically connected to the charge detection circuit 35 and the photoelectric conversion unit 10C. The photoelectric conversion unit 10C of the pixel 24 includes, for example, the above-described photoelectric conversion device 10A or photoelectric conversion device 10B. In the example shown in Fig. 5, the photoelectric conversion unit 10C includes the photoelectric conversion device 10B. The charge storage node 34 accumulates charges generated in the photoelectric conversion unit 10C, and the charge detection circuit 35 detects the charges accumulated in the charge storage node 34. The charge detection circuit 35 provided in the semiconductor substrate 40 may be provided on the semiconductor substrate 40 or may be provided in the semiconductor substrate 40.

As shown in Fig. 4, the imaging apparatus 100 includes a plurality of pixels 24 and peripheral circuits. The imaging apparatus 100 is an organic image sensor realized by a one-chip integrated circuit and includes a pixel array including a plurality of pixels 24 two-dimensionally arrayed.

The plurality of pixels 24 is disposed two-dimensionally, i.e., in the row direction and the column direction, on the semiconductor substrate 40 and form a photosensitive area, i.e., a pixel area. Fig. 4 shows an example in which pixels 24 are disposed in a matrix of two rows and two columns. In Fig. 4, for convenience of illustration, a circuit for setting the sensitivity of the pixels 24 individually (for example, pixel electrode control circuit) is omitted. The imaging apparatus 100 may be a line sensor. In such a case, a plurality of pixels 24 may be disposed one-dimensionally. In the present specification, the row direction and the column direction refer to the directions in which the row and the column extend, respectively. That is, in Fig. 4, the vertical direction on the paper is the column direction, and the horizontal direction is the row direction.

As shown in Figs. 4 and 5, each pixel 24 includes a photoelectric conversion unit 10C, a charge detection circuit 35, and a charge storage node 34 electrically connected to the photoelectric conversion unit 10C and the charge detection circuit 35. The charge detection circuit 35 includes an amplification transistor 21, a reset transistor 22, and an address transistor 23.

The photoelectric conversion unit 10C includes a lower electrode 2 provided as a pixel electrode and an upper electrode 4 provided as a counter electrode facing the pixel electrode. The upper electrode 4 is applied with a predetermined bias voltage through a counter electrode signal line 26.

The lower electrode 2 is a pixel electrode provided to each of the pixels 24, and a plurality of the lower electrodes 2 are arranged in an array. The lower electrode 2 is connected to the gate electrode 21G of the amplification transistor 21, and the signal charges collected by the lower electrode 2 are accumulated in the charge storage node 34 located between the lower electrode 2 and the gate electrode 21G of the amplification transistor 21. In the present embodiment, the signal charge is a hole, but the signal charge may be an electron.

The signal charges accumulated in the charge storage node 34 are applied to the gate electrode 21G of the amplification transistor 21 as a voltage according to the amount of the signal charges. The amplification transistor 21 amplifies this voltage. The amplified voltage is selectively read as a signal voltage by the address transistor 23. The reset transistor 22 is connected to the lower electrode 2 through the source/drain electrode and resets the signal charges accumulated in the charge storage node 34. In other words, the reset transistor 22 resets the potentials of the gate electrode 21G of the amplification transistor 21 and the lower electrode 2.

In order to perform the above-described operation selectively in the plurality of pixels 24, the imaging apparatus 100 includes power supply wiring 31, a vertical signal line 27, an address signal line 36, and a reset signal line 37, and these lines are respectively connected to each of the pixels 24. Specifically, the power supply wiring 31 is connected to the source/drain electrode of the amplification transistor 21, and the vertical signal line 27 is connected to the source/drain electrode of the address transistor 23. The address signal line 36 is connected to the gate electrode 23G of the address transistor 23. The reset signal line 37 is connected to the gate electrode 22G of the reset transistor 22.

The peripheral circuits include a vertical scanning circuit 25, a horizontal signal reading circuit 20, a plurality of column signal processing circuits 29, a plurality of load circuits 28, and a plurality of differential amplifiers 32. The vertical scanning circuit 25 is also referred to as a row scanning circuit. The horizontal signal reading circuit 20 is also referred to as a column scanning circuit. The column signal processing circuit 29 is also referred to as a row signal storage circuit. The differential amplifier 32 is also referred to as a feedback amplifier.

The vertical scanning circuit 25 is connected to the address signal line 36 and the reset signal line 37, selects a plurality of pixels 24 arranged in each row on a row-by-row basis, and performs reading of the signal voltage and resetting of the potential of the lower electrode 2. The power supply wiring 31 functioning as a source follower power supply supplies a predetermined power supply voltage to each pixel 24. The horizontal signal reading circuit 20 is electrically connected to a plurality of column signal processing circuits 29. The column signal processing circuits 29 are electrically connected to the pixels 24 arranged in each of the columns through the vertical signal lines 27 corresponding to the respective columns. The load circuits 28 are electrically connected to the respective vertical signal lines 27. The load circuit 28 and the amplification transistor 21 form a source follower circuit.

The plurality of differential amplifiers 32 are provided so as to correspond to the respective columns. The input terminal on the negative side of the differential amplifier 32 is connected to the corresponding vertical signal line 27. The output terminal of the differential amplifier 32 is connected to the pixel 24 through a feedback line 33 corresponding to each column.

The vertical scanning circuit 25 applies a row selection signal that controls ON and OFF of the address transistor 23 to the gate electrode 23G of the address transistor 23 by the address signal line 36. Consequently, the row as the readout target is scanned and selected. The signal voltage is read out from the pixels 24 in the selected row into the vertical signal line 27. The vertical scanning circuit 25 applies a reset signal that controls ON and OFF of the reset transistor 22 to the gate electrode 22G of the reset transistor 22 through the reset signal line 37. Consequently, the row of pixels 24 as the target of reset operation is selected. The vertical signal line 27 transmits the signal voltage read out from the pixels 24 selected by the vertical scanning circuit 25 to the column signal processing circuit 29.

The column signal processing circuit 29 performs noise suppression signal processing represented by correlated double sampling, analog-digital conversion (AD conversion), and so on.

The horizontal signal reading circuit 20 sequentially reads out signals from a plurality of column signal processing circuits 29 to a horizontal common signal line (not shown).

The differential amplifier 32 is connected to the drain electrode of the reset transistor 22 through the feedback line 33. Accordingly, the differential amplifier 32 receives the output value of the address transistor 23 in the negative terminal when the address transistor 23 and the reset transistor 22 are in a conduction state. The differential amplifier 32 performs feedback operation such that the gate potential of the amplification transistor 21 is a predetermined feedback voltage. On this occasion, the output voltage value of the differential amplifier 32 is 0 V or a positive voltage near 0 V. The feedback voltage means an output voltage of the differential amplifier 32.

As shown in Fig. 5, the pixel 24 includes a semiconductor substrate 40, a charge detection circuit 35, a photoelectric conversion unit 10C, and a charge storage node 34 (see Fig. 4).

The semiconductor substrate 40 may be an insulative substrate provided with a semiconductor layer on the surface on the side where the photosensitive area is formed, for example, a p-type silicon substrate. The semiconductor substrate 40 has impurity areas 21D, 21S, 22D, 22S, and 23S and a device isolation region 41 for electrical separation between pixels 24. The impurity areas 21D, 21S, 22D, 22S, and 23S are, for example, n-type areas. Here, the device isolation region 41 is also provided between the impurity area 21D and the impurity area 22D. Consequently, the signal charges accumulated in the charge storage node 34 are prevented from leaking. The device isolation region 41 is formed by, for example, acceptor ion implantation under predetermined implantation conditions.

The impurity areas 21D, 21S, 22D, 22S, and 23S are, for example, diffusion layers formed in the semiconductor substrate 40. As shown in Fig. 5, the amplification transistor 21 includes impurity areas 21S and 21D and a gate electrode 21G. The impurity areas 21S and 21D function as, for example, the source region and the drain region, respectively, of the amplification transistor 21. A channel region of the amplification transistor 21 is formed between the impurity areas 21S and 21D.

Similarly, the address transistor 23 includes impurity areas 23S and 21S and a gate electrode 23G connected to the address signal line 36. In this example, the amplification transistor 21 and the address transistor 23 share the impurity area 21S and are thereby electrically connected to each other. The impurity area 23S functions as, for example, the source region of the address transistor 23. The impurity area 23S is connected to the vertical signal line 27 shown in Fig. 4.

The reset transistor 22 includes the impurity areas 22D and 22S and a gate electrode 22G connected to the reset signal line 37. The impurity area 22S functions as, for example, the source region of the reset transistor 22. The impurity area 22S is connected to the reset signal line 37 shown in Fig. 4.

An interlayer insulating layer 50 is stacked on the semiconductor substrate 40 so as to cover the amplification transistor 21, the address transistor 23, and the reset transistor 22.

In the interlayer insulating layer 50, a wiring layer (not shown) can be arranged. The wiring layer is formed from, for example, a metal such as copper and can partially include wiring such as the above-described vertical signal line 27. The number of insulating layers in the interlayer insulating layer 50 and the number of wiring layers arranged in the interlayer insulating layer 50 can be arbitrarily set.

In the interlayer insulating layer 50, a contact plug 54 connected to the impurity area 22D of the reset transistor 22, a contact plug 53 connected to the gate electrode 21G of the amplification transistor 21, a contact plug 51 connected to the lower electrode 2, and wiring 52 connecting the contact plug 51, the contact plug 54, and the contact plug 53 are arranged. Consequently, the impurity area 22D of the reset transistor 22 is electrically connected to the gate electrode 21G of the amplification transistor 21.

The charge detection circuit 35 detects the signal charges captured by the lower electrode 2 and outputs a signal voltage. That is, the charge detection circuit 35 read out the charges generated in the photoelectric conversion unit 10C. The charge detection circuit 35 includes the amplification transistor 21, the reset transistor 22, and the address transistor 23 and is formed in the semiconductor substrate 40.

The amplification transistor 21 is formed in the semiconductor substrate 40 and includes impurity areas 21D and 21S that function as a drain region and a source region, respectively, a gate insulating layer 21X formed on the semiconductor substrate 40, and a gate electrode 21G formed on the gate insulating layer 21X.

The reset transistor 22 is formed in the semiconductor substrate 40 and includes impurity areas 22D and 22S that function as a drain region and a source region, respectively, a gate insulating layer 22X formed on the semiconductor substrate 40, and a gate electrode 22G formed on the gate insulating layer 22X.

The address transistor 23 is formed in the semiconductor substrate 40 and includes impurity areas 21S and 23 S that function as a drain region and a source region, respectively, a gate insulating layer 23X formed on the semiconductor substrate 40, and a gate electrode 23G formed on the gate insulating layer 23X. The impurity area 21S is shared by the amplification transistor 21 and the address transistor 23. Consequently, the amplification transistor 21 and the address transistor 23 are connected in series.

The above-described photoelectric conversion unit 10C is arranged on the interlayer insulating layer 50. In other words, in the present embodiment, a plurality of pixels 24 constituting a pixel array is formed on the semiconductor substrate 40. The plurality of pixels 24 two-dimensionally disposed on the semiconductor substrate 40 forms a photosensitive area. The distance between two adjacent pixels 24 (i.e., pixel pitch) may be, for example, about 2 µm.

The photoelectric conversion unit 10C has a structure of the above-described photoelectric conversion device 10A or photoelectric conversion device 10B.

A color filter 60 is provided above the photoelectric conversion unit 10C, and a microlens 61 is provided thereabove. The color filter 60 is formed as, for example, an on-chip color filter by patterning. In formation of the color filter 60, for example, a photosensitive resin in which a dye or a pigment is dispersed is used. The microlens 61 is formed as, for example, an on-chip microlens. In formation of the microlens 61, for example, an ultraviolet sensitive material is used.

The imaging apparatus 100 can be manufactured using a general semiconductor manufacturing process. In particular, when a silicon substrate is used as the semiconductor substrate 40, various silicon semiconductor processes can be used for the manufacturing.

When the color filter 60 and so on are formed in manufacturing of the imaging apparatus 100, for example, heating to about 200°C is performed. Accordingly, since the photoelectric conversion unit 10C includes the photoelectric conversion device 10A or photoelectric conversion device 10B which can suppress deterioration of the device characteristics due to heating, characteristic deterioration of the imaging apparatus 100 to be manufactured can be suppressed.

### EXAMPLES

The photoelectric conversion device and so on to be used in the imaging apparatus according to the present disclosure will now be specifically described by examples, but the present disclosure is by no means limited only to the following examples.

### [Synthesis of material]

### (Synthesis example 1)

A compound represented by the structural formula (16) above, the compound (A-3) below, was synthesized by the following synthesis procedure.

### (1) Synthesis of compound (A-1)

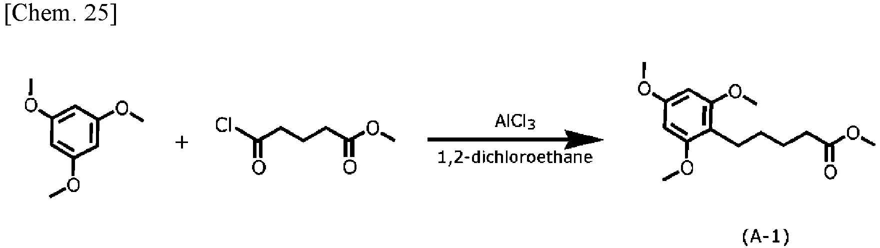

To a three-neck 200-mL reaction vessel purged with argon, 9.2 g of aluminum chloride and 50 mL of 1,2-dichloroethane were added, and the reaction vessel was placed in a water bath containing ice water and was cooled. To the cooled reaction vessel, 7.57 g of 1,3,5-trimethoxybenzene was further added, and the reaction solution was stirred for 5 minutes. To the reaction vessel, 4.94 g of methyl 5-chloro-5-oxopentanoate was further added, the reaction vessel was taken out from the water bath, and the reaction solution was stirred at room temperature for 22 hours.

One hundred grams of ice and 50 mL of dichloromethane were added to the reaction solution to obtain an organic layer and a water layer. The water layer was extracted with 50 mL of dichloromethane three times, and the extract and the separated organic layer were concentrated together to obtain a crude product. Fifty milliliters of toluene was added to the obtained crude product, and the crude product was purified by silica gel column chromatography . In the silica gel column chromatography, the low-polar component was first separated with an eluent of toluene only, and the target component (A-1) above was then extracted with an eluent of toluene : ethyl acetate =1:1. The amount of compound (A-1) was 7.7 g, and the yield was 86%.

### (2) Synthesis of compound (A-2)

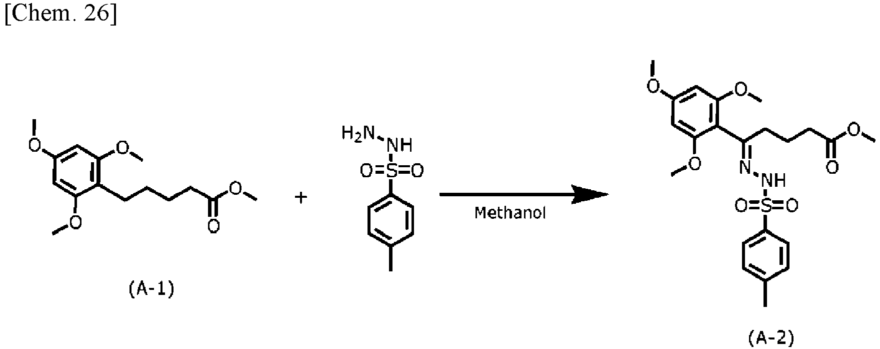

To a three-neck 200-mL reaction vessel purged with argon, 4.5 g of the compound (A-1) synthesized above and 25 mL of methanol were added, followed by stirring. Furthermore, 3.4 g of p-toluenesulfonyl hydrazide was added thereto, and the reaction solution was heated and refluxed at 65°C for 7 hours. The reaction solution was cooled to room temperature and then concentrated, and the concentrate was cooled overnight in a refrigerator. Fifty milliliters of cold methanol cooled in a refrigerator overnight was added to the cooled concentrate to precipitate the solid component, and the precipitated solid component was washed with 2 mL of cold methanol twice. The washed solid component was dried under reduced pressure at room temperature for 3 hours to obtain the target compound (A-2) above. The amount of the compound (A-2) was 4.7 g, and the yield was 67%.

### (3) Synthesis of compound (A-3)

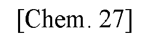

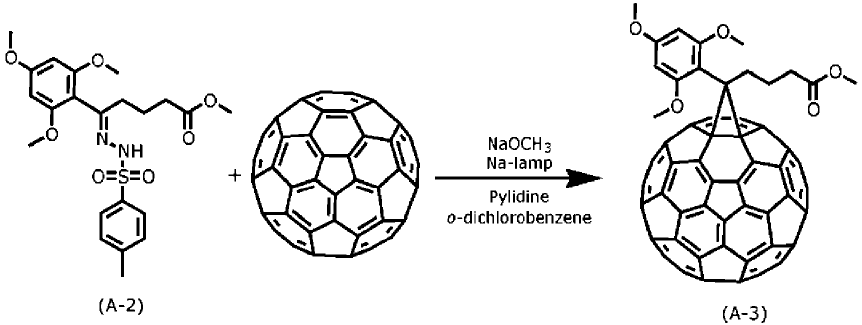

To a three-neck 200-mL reaction vessel purged with argon (hereinafter, referred to as first reaction vessel), 449 mg of the compound (A-2) synthesized above, 25 mL of dehydrated pyridine, and 60 mg of sodium methoxide were added, and the reaction solution was stirred for 30 minutes. To a three-neck 200-mL reaction vessel purged with argon (hereinafter, referred to as second reaction vessel), 735 mg of C60 fullerene and 75 mL of deoxygenated o-dichlorobenzene were added, and the reaction solution was stirred. The whole reaction solution in the second reaction vessel was added to the first reaction vessel, and the reaction solution in the first reaction vessel was subjected to bubbling with argon for 30 minutes. The first reaction vessel was irradiated with a 150 W sodium lamp, the distance between the first reaction vessel and the 150 W sodium lamp was adjusted such that the temperature of the reaction solution was 86°C, and the reaction solution was stirred for 12 hours. The reaction solution was cooled to room temperature and was then concentrated to obtain a crude product. The crude product was purified by silica gel column chromatography using o-dichlorobenzene as the eluent, and the obtained purified product was further reprecipitated with methanol. The obtained precipitate was dried under reduced pressure at 40°C for 24 hours to obtain the target compound (A-3) above. The amount of the compound (A-3) was 432 mg, and the yield was 39%.

The compound was identified by ¹HNMR (proton nuclear magnetic resonance). The results are shown below:
¹HNMR (400 MHz, o-C₆D₄Cl₂): δ (ppm) = 6.21 (s, 2H), 3.76 (s, 6H), 3.63 (s, 3H), 3.51 (s, 3H), 2.90 (t, 2H), 2.51 (t, 2H), 2.26 (m, 2H).

The results above demonstrate that the compound (A-3) can be obtained by the synthesis procedure above.

A fullerene derivative having a substituent that binds to the phenyl group of the phenylbutyric acid methyl ester portion is different from that of the compound (A-3) can be synthesized by using a starting material in which the substituent of the benzene ring of 1,3,5-trimethoxybenzene as the starting material of the compound (A-3) was changed.

### (Synthesis example 2)

A compound represented by the structural formula (9) above, the compound (A-6) below, was synthesized by the following synthesis procedure.

### (1) Synthesis of compound (A-5)

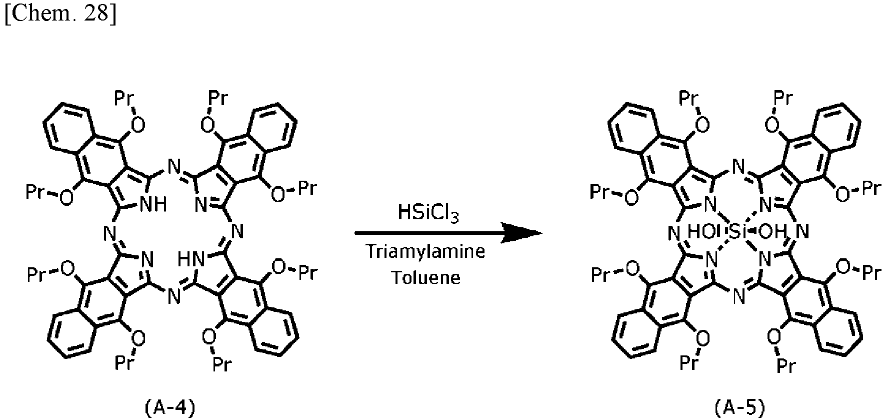

To a 50-mL reaction vessel purged with argon, 50 mg of the compound (A-4), 5 mL of triamylamine, and 25 mL of dehydrated toluene were added, and 0.5 mL of HSiCl₃ was further added thereto, followed by heating and stirring at 90°C for 24 hours.

The reaction solution was allowed to cool to room temperature, and 20 mL of distilled water was added to the reaction solution, followed by stirring for 1 hour. The reaction solution was extracted with 60 mL of toluene four times. The extracted organic layer was washed with distilled water, and the organic layer was concentrated to obtain 48 mg of a crude product. The obtained crude product was purified with a neutral alumina column to obtain the target compound (A-5) as a brown solid. The amount of the compound (A-5) was 25 mg, and the yield was 49%.

### (2) Synthesis of compound (A-6)

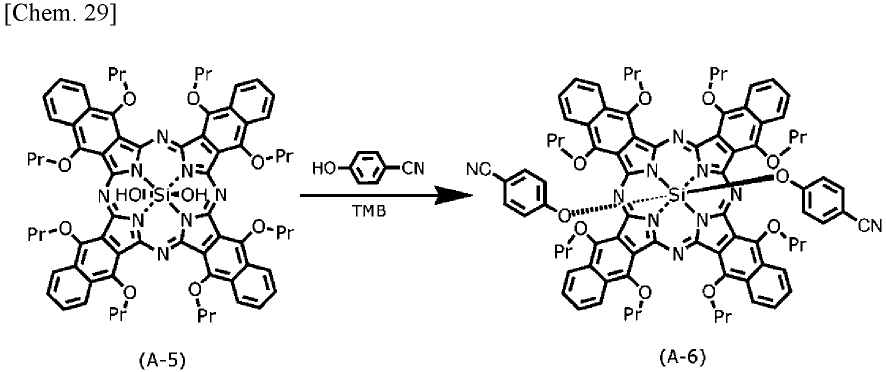

To a 200 mL reaction vessel purged with argon, 0.75 g of the compound (A-5) synthesized above and 0.91 g of 4-cyanophenol were added, and they were dissolved in 30 mL of 1,2,4-trimethylbenzene (TMB), followed by heating and refluxing at 180°C for 3 hours. The reaction solution was cooled to room temperature, and 50 mL of heptane was then added to the reaction solution to precipitate the solid component. The precipitated solid component was collected by filtration. The collected solid component was purified by silica gel column chromatography using toluene : ethyl acetate = 1 : 1 as the eluent. The obtained purified product was further reprecipitated with heptane. The obtained precipitate was dried under reduced pressure at 100°C for 3 hours to obtain the target compound (A-6). The amount of the compound (A-6) was 557 mg, the yield was 74%.

The obtained compound was identified by ¹HNMR, MALDI-TOF-MS (matrix assisted laser desorption-ionization time of flight mass spectrometry). The results are shown below:
¹HNMR (400 MHz, C₆D₆): δ (ppm) = 9.11 (8H), 7.58 (8H), 5.58 (4H), 5.06 (16H), 3.70 (4H), 2.24 (16H), 1.11 (24H);
MALDI-TOF-MS measured value: m/z = 1441.82 (M⁺).

The chemical formula of the compound (A-6) is C₈₆H₈₀N₁₀O₁₀Si, and exact mass is 1441.82.

The results above demonstrate that the compound (A-6) can be obtained by the synthesis procedure above.

### (Synthesis example 3)

A compound represented by the structural formula (10) above, the compound (A-7) below, was synthesized by the following synthesis procedure.

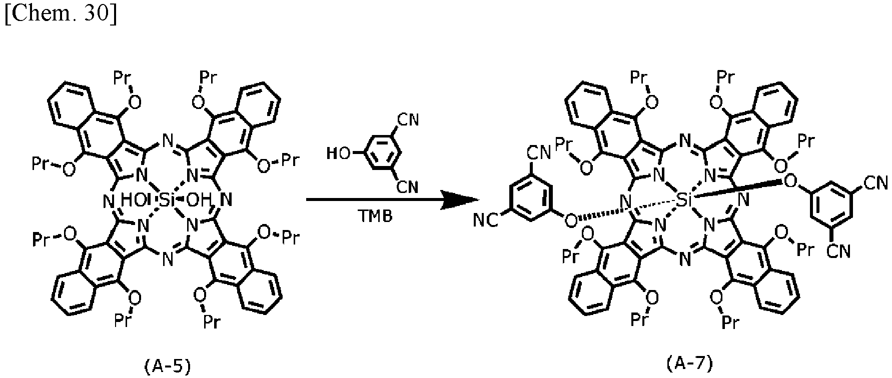

To a 200-mL reaction vessel purged with argon, 0.64 g of the compound (A-5) synthesized in the "(1) Synthesis of compound (A-5)" of Synthesis example 2 above and 1.13 g of 3,5-dicyanophenol were added, and they were dissolved in 40 mL of 1,2,4-trimethylbenzene (TMB), followed by heating and refluxing at 180°C for 5 hours. The reaction solution was cooled to room temperature, and 50 mL of heptane was added to the reaction solution to precipitate the solid component. The precipitated solid component was collected by filtration. The collected solid component was purified by silica gel column chromatography using dichloromethane as the eluent. The purified product was further reprecipitated with heptane. The obtained precipitate was dried under reduced pressure at 100°C for 3 hours to obtain the target compound (A-7). The amount of the compound (A-7) was 528 mg, the yield was 68%.

The obtained compound was identified by ¹HNMR, MALDI-TOF-MS. The results are shown below:
¹HNMR (400 MHz, C₆D₆): δ (ppm) = 9.08 (8H), 7.55 (8H), 5.22 (2H), 4.08 (4H), 2.36 (16H), 1.23 (24H);
MALDI-TOF-MS measured value: m/z = 1491.83 (M⁺).

The chemical formula of the compound (A-7) is C₈₈H₇₈N₁₂O₁₀Si, and the exact mass is 1491.75.

The results above demonstrate that the compound (A-7) can be obtained by the synthesis procedure above.

### (Synthesis example 4)

A compound represented by the structural formula (8) above, the compound (A-10) below, was synthesized by the following synthesis procedure.

### (1) Synthesis of compound (A-9)

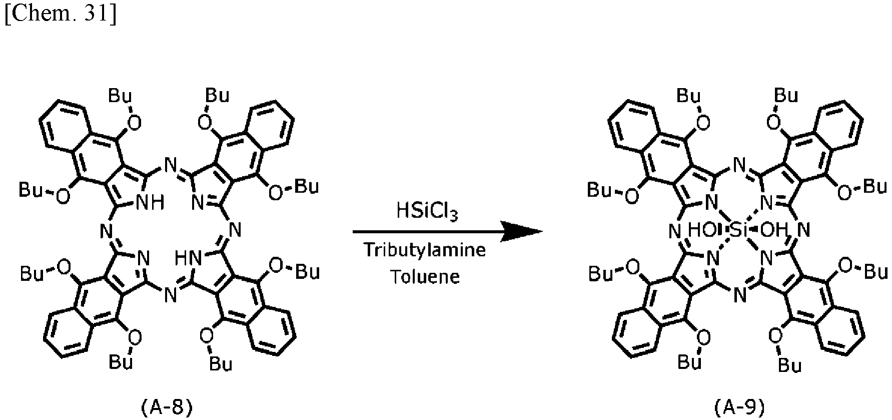

To a 1000-mL reaction vessel purged with argon, 0.95 g of the compound (A-8) above, 92 mL of tributylamine, and 550 mL of dehydrated toluene were added, and 3.7 mL of HSiCl₃ was further added thereto, followed by heating and stirring at 80°C for 24 hours. Subsequently, the reaction solution was allowed to cool to room temperature, 3.7 mL of HSiCl₃ was added thereto, followed by heating and stirring at 80°C for 24 hours. Subsequently, the reaction solution was allowed to cool to room temperature, 1.9 mL of HSiCl₃ was added thereto, followed by heating and stirring at 80°C for 24 hours.

The reaction solution was allowed to cool to room temperature, and 360 mL of distilled water was added to the reaction solution, followed by stirring for 1 hour. Furthermore, 180 mL of triethylamine was added thereto, followed by extraction with 100 mL of toluene four times. The extracted organic layer was washed with distilled water, and the washed organic layer was concentrated to obtain 1.54 g of a crude product. The obtained crude product was purified with a neutral alumina column to obtain the target compound (A-9) as a brown solid. The amount of the compound (A-9) was 0.53 g, and the yield was 50%.

### (2) Synthesis of compound (A-10)

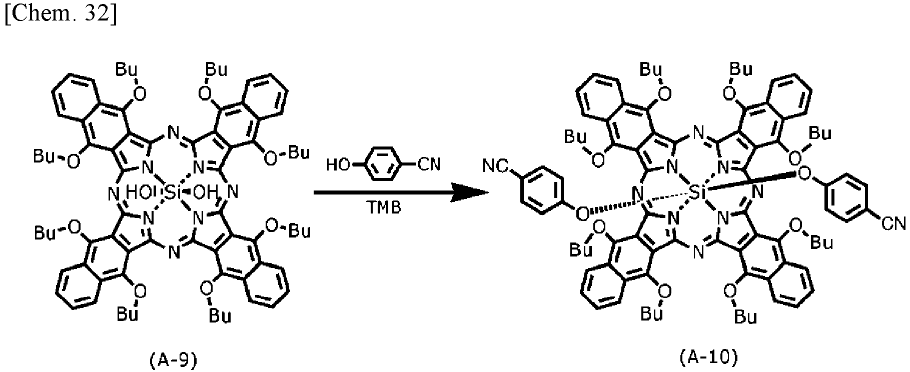

To a 200-mL reaction vessel purged with argon, 0.2 g of the compound (A-9) synthesized above and 0.88 g of 4-cyanophenol were added, and they were dissolved in 15 mL of 1,2,4-trimethylbenzene (TMB), followed by heating and refluxing at 180°C for 3 hours. After cooling to room temperature, 30 mL of ethanol was added to the reaction solution to precipitate the solid component, and the precipitated solid component was collected by filtration. The collected solid component was purified by silica gel column chromatography using toluene as the eluent. The obtained purified product was further reprecipitated with methanol, and the obtained precipitate was dried under reduced pressure at 100°C for 3 hours to obtain the target compound (A-10). The amount of the compound (A-10) was 159 mg, and the yield was 69%.

The obtained compound was identified by ¹HNMR, MALDI-TOF-MS. The results are shown below:
¹HNMR (400 MHz, C₆D₆): δ (ppm) = 9.14 (8H), 7.60 (8H), 5.65 (4H), 5.11 (16H), 3.75 (4H), 2.28 (16H), 1.62 (16H), 0.98 (24H),
MALDI-TOF-MS measured value: m/z = 1553.95 (M⁺).

The chemical formula of the compound (A-10) is C₉₄H₉₆N₁₀O₁₀Si, and the exact mass is 1553.71.

The results above demonstrate that the compound (A-10) can be obtained by the synthesis procedure above.

### (Synthesis example 5)

A compound represented by the structural formula (17) above, the compound (A-13) below, was synthesized by the following synthesis procedure.

### (1) Synthesis of compound (A-11)

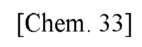

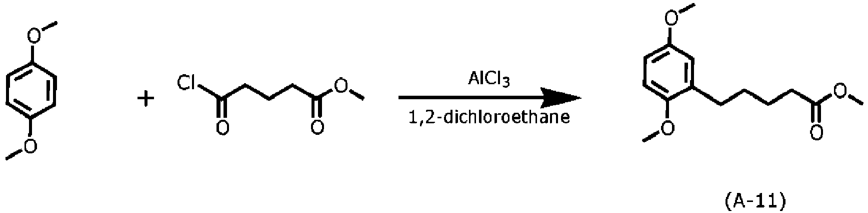

To a three-neck 200-mL reaction vessel purged with argon, 9.04 g of aluminum chloride and 50 mL of 1,2-dichloroethane were added, and the reaction vessel was placed in a water bath containing ice water and was cooled. Furthermore, 6.29 g of p-trimethoxybenzene was added to the cooled reaction vessel, and the reaction solution was stirred for 5 minutes. Furthermore, 5.10 g of methyl 5-chloro-5-oxopentanoate was added to the reaction vessel, the reaction vessel was taken out from the water bath, and the reaction solution was stirred at room temperature for 20 hours.

Twenty grams of ice, 50 mL of water, and 50 mL of dichloromethane were added to the reaction solution to obtain an organic layer and a water layer. The water layer was extracted with 50 mL of dichloromethane three times, and the extract and the separated organic layer were concentrated together to obtain a crude product. Fifty milliliters of toluene was added to the obtained crude product, and the crude product was purified by silica gel column chromatography . In the silica gel column chromatography, the low-polar component was first separated with an eluent of toluene only, and the target component (A-11) above was then extracted with an eluent of toluene : ethyl acetate = 1 : 1. The amount of compound (A-11) was 6.4 g, and the yield was 78%.

### (2) Synthesis of compound (A-12)

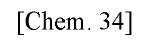

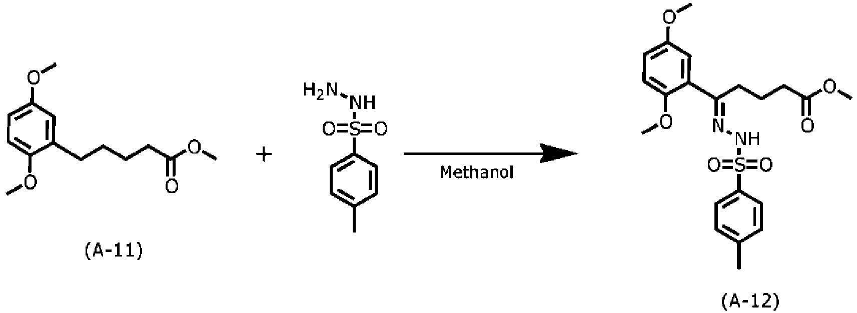

To a three-neck 200-mL reaction vessel purged with argon, 3.02 g of the compound (A-11) synthesized above and 25 mL of methanol were added, followed by stirring. Furthermore, 2.57 g of p-toluenesulfonyl hydrazide was added thereto, and the reaction solution was heated and refluxed at 65°C for 7 hours. The reaction solution was cooled to room temperature and was then concentrated, and the concentrate was cooled overnight in a refrigerator. Fifty milliliters of cold methanol cooled in a refrigerator overnight was added to the cooled concentrate to precipitate the solid component, and the precipitated solid component was washed with 2 mL of cold methanol twice. The washed solid component was dried under reduced pressure at room temperature for 3 hours to obtain the target compound (A-12) above. The amount of the compound (A-12) was 2.0 g, and the yield was 41%.

### (3) Synthesis of compound (A-13)

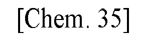

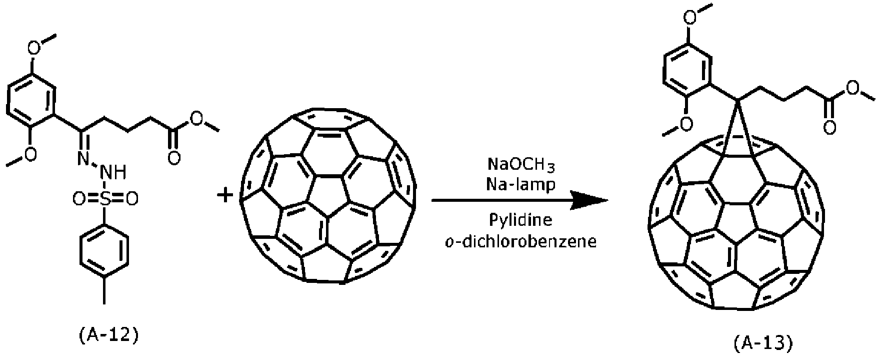

To a three-neck 200-mL reaction vessel purged with argon (hereinafter, referred to as first reaction vessel), 711 mg of the compound (A-12) synthesized above, 20 mL of dehydrated pyridine, and 80 mg of sodium methoxide were added, and the reaction solution was stirred for 30 minutes. To another three-neck 200-mL reaction vessel purged with argon (hereinafter, referred to as second reaction vessel), 1.2 g of C60 fullerene and 100 mL of deoxygenated o-dichlorobenzene were added, and the reaction solution was stirred. The whole reaction solution in the second reaction vessel was added to the first reaction vessel, and the reaction solution in the first reaction vessel was subjected to bubbling with argon for 30 minutes. The first reaction vessel was irradiated with a 180 W sodium lamp, the distance between the first reaction vessel and the 180 W sodium lamp was adjusted such that the temperature of the reaction solution was from 95°C to 105°C, and the reaction solution was stirred for 7 hours. The reaction solution was cooled to room temperature and was then concentrated to obtain a crude product. The crude product was purified by silica gel column chromatography using o-dichlorobenzene as the eluent, and the obtained purified product was further reprecipitated with a solution mixture of methanol and acetone of 1 : 1. The obtained precipitate was suspended in and washed with acetone twice and was then dried under reduced pressure at 60°C for 4 hours to obtain the target compound (A-13) above. The amount of the compound (A-13) was 473 mg, and the yield was 30%.

The compound was identified by ¹HNMR. The results are shown below:

¹HNMR (400 MHz, o-C₆D₄Cl₂): δ (ppm) = 7.41 (s, 1H), 6.81 (s, 1H), 6.75 (s, 1H), 3.80 (s, 3H), 3.62 (s, 3H), 3.49 (s, 3H), 2.86 (t, 2H), 2.39 (t, 2H), 2.18 (m, 2H).

The results above demonstrate that the compound (A-13) can be obtained by the synthesis procedure above.

### (Synthesis example 6)

A compound represented by the structural formula (18) above, the compound (A-16) below, was synthesized by the following synthesis procedure.

### (1) Synthesis of compound (A-14)

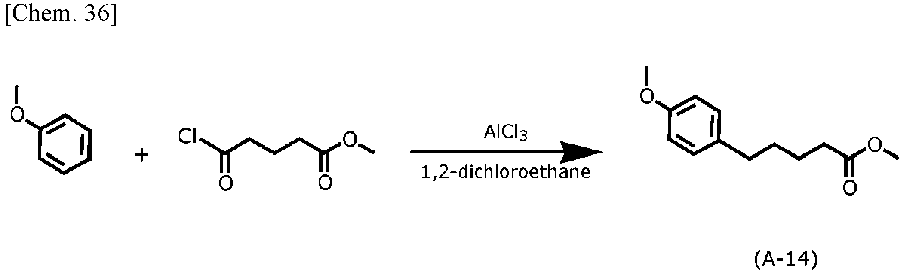

To a three-neck 200-mL reaction vessel purged with argon, 8.1 g of aluminum chloride and 50 mL of 1,2-dichloroethane were added, and the reaction vessel was placed in a water bath containing ice water and was cooled. Furthermore, 4.3 g of anisole was added to the cooled reaction vessel, the reaction solution was stirred for 5 minutes. Furthermore, 4.4 g of methyl 5-chloro-5-oxopentanoate was added to the reaction vessel, the reaction vessel was taken out from the water bath, and the reaction solution was stirred at room temperature for 15 hours.

Twenty grams of ice, 50 mL of water, and 50 mL of dichloromethane were added to the reaction solution to obtain an organic layer and a water layer. The water layer was extracted with 50 mL of dichloromethane three times, and the extract and the separated organic layer were concentrated together to obtain a crude product. Fifty milliliters of toluene was added to the obtained crude product, and the crude product was purified by silica gel column chromatography. In the silica gel column chromatography, the low-polar component was first separated with an eluent of toluene only, followed by purification with an eluent of toluene : ethyl acetate = 1 : 1. The obtained purified product was further suspended in and washed with cold heptane. The suspended washed solid component was dried under reduced pressure at 40°C for 3 hours to obtain the target compound (A-14). The amount of the compound (A-14) was 5.5 g, and the yield was 90%.

### (2) Synthesis of compound (A-15)

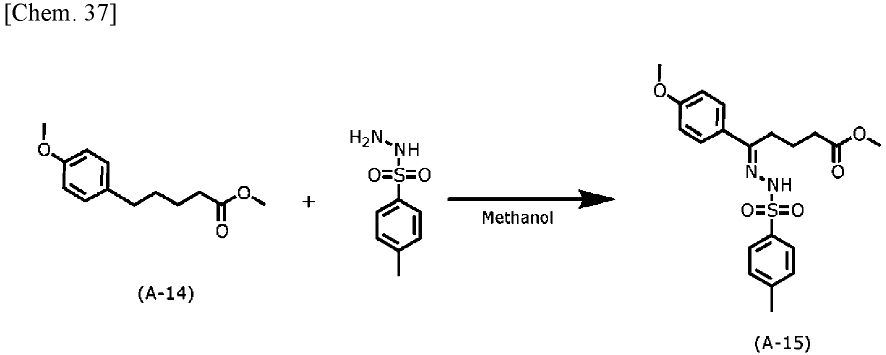

To a three-neck 200-mL reaction vessel purged with argon, 4.4 g of the compound (A-14) synthesized above and 40 mL of methanol were added, followed by stirring. Furthermore, 4.2 g of p-toluenesulfonyl hydrazide was added thereto, and the reaction solution was heated and refluxed at 65°C for 4 hours. The reaction solution was cooled to room temperature and was then concentrated, and the concentrate was cooled in a refrigerator overnight. Fifty milliliters of cold methanol cooled in a refrigerator overnight was added to the cooled concentrate to precipitate the solid component, and the precipitated solid component was washed with 5 mL of cold methanol twice. The washed solid component was dried under reduced pressure at 50°C for 3 hours to obtain the target compound (A-15). The amount of the compound (A-15) was 6.3 g, and the yield was 84%.

### (3) Synthesis of compound (A-16)

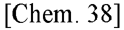

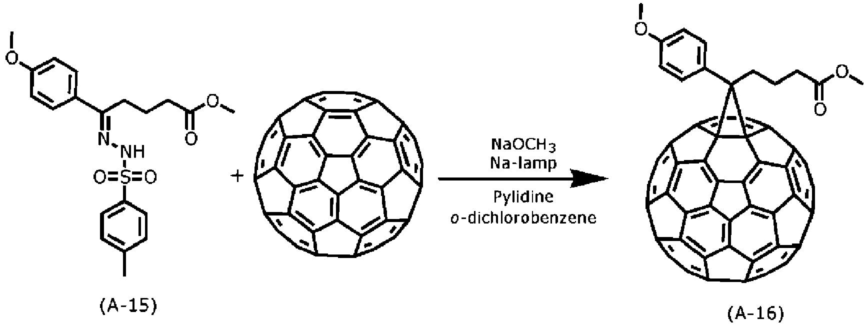

To a three-neck 200-mL reaction vessel purged with argon (hereinafter, referred to as first reaction vessel), 611 mg of the compound (A-15) synthesized above, 20 mL of dehydrated pyridine, and 80 mg of sodium methoxide were added, and the reaction solution was stirred for 30 minutes. To another three-neck 200-mL reaction vessel purged with argon (hereinafter, referred to as second reaction vessel), 1.0 g of C60 fullerene and 100 mL of deoxygenated o-dichlorobenzene were added, and the reaction solution was stirred. The whole reaction solution in the second reaction vessel was added to the first reaction vessel, and the reaction solution in the first reaction vessel was subjected to bubbling with argon for 30 minutes. The first reaction vessel was irradiated with a 180 W sodium lamp, the distance between the first reaction vessel and the 180 W sodium lamp was adjusted such that the temperature of the reaction solution was 86°C, and the reaction solution was stirred for 7 hours. The reaction solution was cooled to room temperature and was then concentrated to obtain a crude product. The crude product was dissolved in o-dichlorobenzene, followed by filtration. The solution after filtration was then purified by silica gel column chromatography using o-dichlorobenzene as the eluent, and the obtained purified product was further reprecipitated with a solution mixture of methanol and acetone of 1 : 1. The obtained precipitate was suspended in and washed with acetone twice and was then dried under reduced pressure at 60°C for 4 hours to obtain the target compound (A-16). The amount of the compound (A-16) was 575 mg, and the yield was 40%.

The compound was identified by ¹HNMR. The results are shown below:

¹HNMR (400 MHz, o-C₆D₄Cl₂): δ (ppm) = 7.23 (d, 2H), 3.58 (s, 3H), 3.51 (s, 3H), 2.80 (t, 2H), 2.40 (t, 2H), 2.15 (m, 2H).

It is inferred that the 2H peak of the phenyl portion of a methyl phenylbutyrate framework overlaps with the peak of o-C₆D₄Cl₂.

The results above demonstrate that the compound (A-16) can be obtained by the synthesis procedure above.

### (Synthesis example 7)

A compound represented by the structural formula (19) above, the compound (A-19) below, was synthesized by the following synthesis procedure.

### (1) Synthesis of compound (A-17)

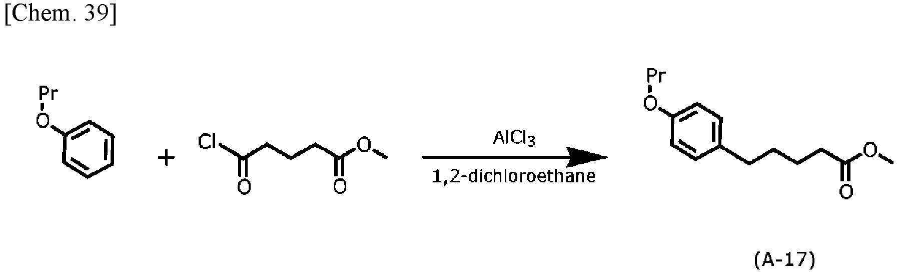

To a three-neck 200-mL reaction vessel purged with argon, 7.2 g of aluminum chloride and 50 mL of 1,2-dichloroethane were added, and the reaction vessel was placed in a water bath containing ice water and was cooled. Furthermore, 4.8 g of propyoxybenzene was added to the cooled reaction vessel, and the reaction solution was stirred for 5 minutes. Furthermore, 3.9 g of methyl 5-chloro-5-oxopentanoate was added to the reaction vessel, the reaction vessel was taken out from the water bath, and the reaction solution was stirred at room temperature for 15 hours.

Twenty grams of ice, 50 mL of water, and 50 mL of dichloromethane were added to the reaction solution to obtain an organic layer and a water layer. The water layer was extracted with 50 mL of dichloromethane three times, and the extract and the separated organic layer were concentrated together to obtain a crude product. Fifty milliliters of toluene was added to the obtained crude product, and the crude product was purified by silica gel column chromatography. In the silica gel column chromatography, the low-polar component was first separated with an eluent of toluene only, followed by purification with an eluent of toluene : ethyl acetate = 1 : 1. The obtained purified product was further suspended in and washed with cold heptane. The suspended washed solid component was dried under reduced pressure at room temperature for 3 hours to obtain the target compound (A-17). The amount of the compound (A-17) was 4.2 g, and the yield was 69%.

### (2) Synthesis of compound (A-18)

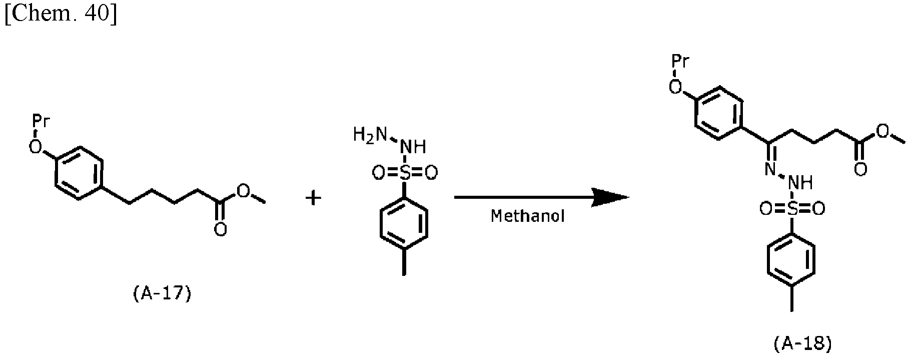

To a three-neck 200-mL reaction vessel purged with argon, 4.1 g of the compound (A-17) synthesized above and 50 mL of methanol were added, followed by stirring. Furthermore, 2.9 g of p-toluenesulfonyl hydrazide was added thereto, and the reaction solution was heated and refluxed at 65°C for 4 hours. The reaction solution was cooled to room temperature and was then concentrated, and the concentrate was cooled in a refrigerator overnight. Fifty milliliters of cold methanol cooled in a refrigerator overnight was added to the cooled concentrate to precipitate the solid component, and the precipitated solid component was washed with 2 mL of cold methanol twice. The washed solid component was dried under reduced pressure at room temperature for 3 hours to obtain the target compound (A-18). The amount of the compound (A-18) was 6.1 g, and yield was 91%.

### (3) Synthesis of compound (A-19)

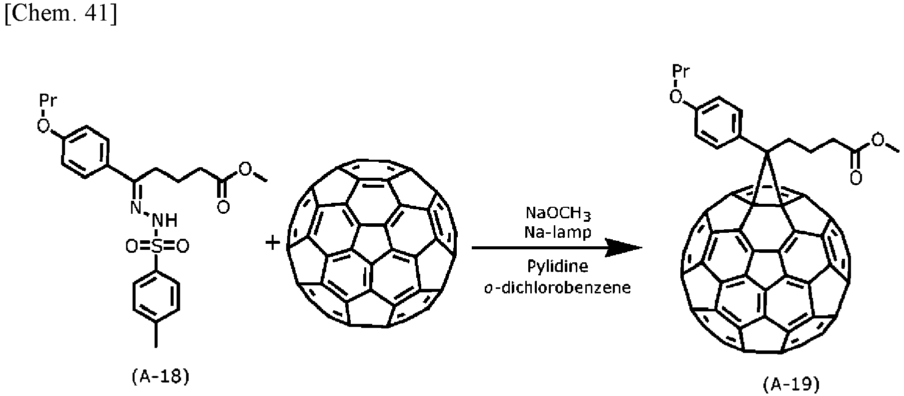

To a three-neck 200-mL reaction vessel purged with argon (hereinafter, referred to as first reaction vessel), 653 mg of the compound (A-18) synthesized above, 20 mL of dehydrated pyridine, and 80 mg of sodium methoxide were added, and the reaction solution was stirred for 30 minutes. To another three-neck 200-mL reaction vessel purged with argon (hereinafter, referred to as second reaction vessel), 1.0 g of C60 fullerene and 100 mL of deoxygenated o-dichlorobenzene were added, and the reaction solution was stirred. The whole reaction solution in the second reaction vessel was added to the first reaction vessel, and the reaction solution in the first reaction vessel was subjected to bubbling with argon for 30 minutes. The first reaction vessel was irradiated with a 180 W sodium lamp, the distance between the first reaction vessel and the 150 W sodium lamp was adjusted such that the temperature of the reaction solution was 86°C, and the reaction solution was stirred for 7 hours. The reaction solution was cooled to room temperature and was then concentrated to obtain a crude product. The crude product was dissolved in o-dichlorobenzene, followed by filtration. The solution after filtration was then purified by silica gel column chromatography using o-dichlorobenzene as the eluent, and the obtained purified product was further reprecipitated with a solution mixture of methanol and acetone of 1 : 1. The obtained precipitate was suspended in and washed with acetone twice and was then dried under reduced pressure at 60°C for 4 hours to obtain the target compound (A-19). The amount of the compound (A-19) was 386 mg, and the yield was 29%.

The compound was identified by ¹HNMR. The results are shown below:

¹HNMR (400 MHz, CDCl₃): δ (ppm) = 7.81 (d, 2H), 7.04 (s, 2H), 4.00 (t, 2H), 3.67 (s, 3H), 2.87 (t, 2H), 2.52 (t, 2H), 2.18 (m, 2H), 1.86 (q, 2H), 1.08 (t, 3H).

The results above demonstrate that the compound (A-19) can be obtained by the synthesis procedure above.

### [Photoelectric conversion device]

The photoelectric conversion device according to the present disclosure will now be more specifically described by showing Example 1 and Comparative Example 1.

### (Example 1)

### <Production of photoelectric conversion device>

A photoelectric conversion device was produced by the following procedure. The production of the photoelectric conversion device was entirely performed in a nitrogen atmosphere.

A glass substrate with a thickness of 0.7 mm provided with an ITO film with a thickness of 150 nm as a lower electrode on one of the main surfaces was prepared. In a glove box of a nitrogen atmosphere, a buffer layer functioning as an electron blocking layer was formed by applying an o-xylene solution of 10 mg/mL of VNPB (N4,N4'-di(naphthalen-1-yl)-N4,N4'-bis(4- vinylphenyl)biphenyl-4,4'-diamine, manufactured by Luminescence Technology Corp.) onto the lower electrode by spin coating. The film formed was heated using a hot plate at 200°C for 50 minutes to crosslink the VNPB, resulting in insolubilization of the buffer layer functioning as an electron blocking layer. Subsequently, a mixture film, which will become a photoelectric conversion layer, was formed by spin coating using a chloroform mixture solution containing the compound (A-6) as a donor organic compound and the compound (A-3) as an acceptor organic compound. The thickness of the mixture film obtained at this time was about 175 nm. The weight ratio of the compound (A-6) and the compound (A-3) in the chloroform mixture solution was 1 : 9.

Furthermore, a film of chloroaluminum phthalocyanine (ClAlPc) was formed as a buffer layer functioning as a hole-blocking layer with a thickness of 30 nm by a vacuum evaporation method through a metal shadow mask.

Subsequently, an ITO film was formed as an upper electrode with a thickness of 30 nm on the buffer layer functioning as a hole-blocking layer by sputtering. Consequently, a photoelectric conversion device of Example 1 was obtained. In Example 1, the methoxy group having dipole-dipole interaction with the propyoxy group of the compound (A-6) as the donor organic compound binds to the aromatic portion of the compound (A-3) as the acceptor organic compound. That is, an attractive force due to the dipole-dipole interaction between the donor organic compound and the acceptor organic compound is likely to occur.

### <Characteristic evaluation of photoelectric conversion device>

As characteristic evaluation of the obtained photoelectric conversion device, the dark current and the photoelectric conversion efficiency were evaluated by the following methods. In the characteristic evaluation, a produced photoelectric conversion device was heated using a hot plate in a glove box at 150°C, 170°C, and 200°C each for 10 minutes, and the characteristics of the photoelectric conversion device were evaluated after heating at each temperature.

### (A) Measurement of photoelectric conversion efficiency

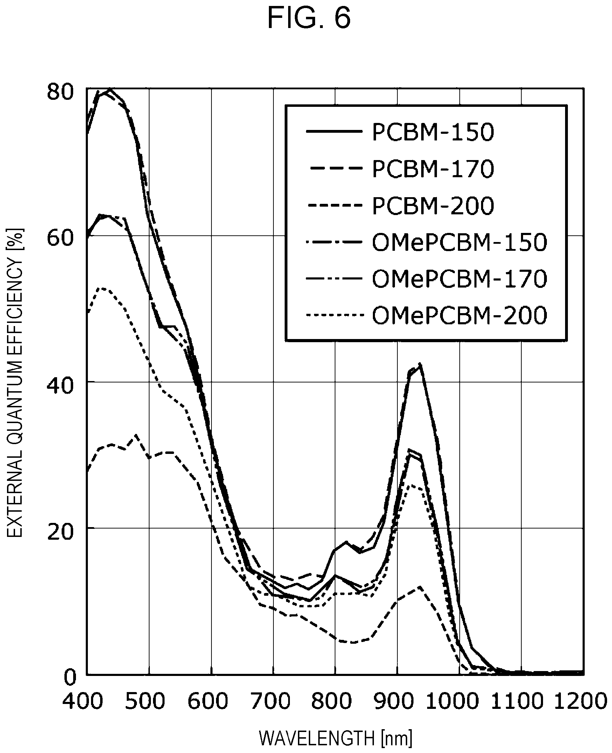

The photoelectric conversion efficiency of the obtained photoelectric conversion device was measured. Specifically, the photoelectric conversion device was introduced in a measurement jig that can be sealed in a glove box in a nitrogen atmosphere, and the external quantum efficiency was measured using a long wavelength responding spectral sensitivity measuring apparatus (manufactured by Bunkoukeiki Co., Ltd., CEP-25RR) under a voltage condition of 10 V. The measurement results are shown in Fig. 6. In the legend in Fig. 6, the numerical value is the temperature at which the photoelectric conversion device is heated, "OMePCBM" indicates that the photoelectric conversion device is that in Example 1, and "PCBM" indicates that the photoelectric conversion device is that in Comparative Example 1.

### (B) Measurement of dark current

The dark current in the obtained photoelectric conversion device was measured. The measurement was performed using B1500A Semiconductor Device Parameter Analyzer (manufactured by Keysight Technologies) in a glove box in a nitrogen atmosphere. The value dark current when a voltage of 10 V was applied is shown in Table 1.

### (Comparative Example 1)

A photoelectric conversion device was produced as in Example 1 except that in the formation of the photoelectric conversion layer, [60] PCBM was used instead of the compound (A-3) as the acceptor organic compound. In Comparative Example 1, the substituent having dipole-dipole interaction with the propyoxy group of the compound (A-6) as the donor organic compound does not bind to the aromatic portion of [60] PCBM as the acceptor organic compound. That is, an attractive force due to the dipole-dipole interaction between the donor organic compound and the acceptor organic compound is unlikely to occur.

The characteristics of the obtained photoelectric conversion device were evaluated as in Example 1. The results of characteristic evaluation are shown in Fig. 6 and Table 1.

**[Table 1]**

| Heating temperature [°C] | Dark current @ 10 V [mA/cm²] | |
|---|---|---|
| | Example 1 | Comparative Example 1 |
| 150 | 1.5 × 10⁻⁷ | 2.3 × 10⁻⁷ |
| 170 | 5.1 × 10⁻⁸ | 7.3 × 10⁻⁸ |
| 200 | 2.3 × 10⁻⁷ | 6.8 × 10⁻⁷ |

As shown in Fig. 6, the external quantum efficiency of the photoelectric conversion device of Comparative Example 1 was drastically reduced when heated to 200°C (PCBM-200 of the legend) compared to when heated to 150°C and 170°C (PCBM-150 and PCBM-170 in the legend). In contrast, in the photoelectric conversion device of Example 1, the reduction in the external quantum efficiency was suppressed compared to the photoelectric conversion device of Comparative Example 1 when heated to 150°C or 170°C (OMePCBM-150 and OMePCBM-170 in the legend) and even when heated to 200°C (OMePCBM-200 in the legend).

As shown in Table 1, in the photoelectric conversion device of Comparative Example 1, the dark current when heated to 200°C was more than two times that when heated to 150°C or 170°C. In contrast, in the photoelectric conversion device of Example 1, the increase in the dark current was suppressed compared to the photoelectric conversion device of Comparative Example 1 when heated to 150°C or 170°C and even when heated to 200°C.

It is inferred that these results are caused by suppression of aggregation and so on of the donor organic compound molecules and of the acceptor organic compound molecules in the photoelectric conversion device of Example 1 by the dipole-dipole interaction between the substituent of the donor organic compound and the substituent of the acceptor organic compound, even when the photoelectric conversion device is heated. In contrast, it is inferred that in the photoelectric conversion device of Comparative Example 1, the donor organic compound molecules and the acceptor organic compound molecules aggregate by heating to deteriorate the device characteristics.

The results above demonstrated that deterioration of the device characteristics by exposure to high temperature can be suppressed in a photoelectric conversion device including a photoelectric conversion layer constituted of a bulk heterojunction layer containing a donor organic compound and an acceptor organic compound in which a substituent having dipole-dipole interaction with the substituent of the donor organic compound is bound to the aromatic portion.

The photoelectric conversion device and imaging apparatus according to the present disclosure have been described based on embodiments and examples, but the present disclosure is not limited to these embodiments and examples. Unless departing from the scope of the present disclosure, embodiments and examples with various modifications concerned by those skilled in the art and other aspects constructed by combining some of the components in the embodiments and examples are also included in the scope of the present disclosure.

The photoelectric conversion devices according to the present disclosure may be utilized in solar cells by extracting charges generated by light as energy.

### Industrial Applicability

The photoelectric conversion device and imaging apparatus according to the present disclosure can be applied to an image sensor or the like, for example, an image sensor that may be exposed to high temperature.

### Reference Signs List

1 supporting substrate
2 lower electrode
3 photoelectric conversion layer
3A donor organic semiconductor
3B acceptor organic semiconductor
4 upper electrode
5, 6 buffer layer
10A, 10B photoelectric conversion device
10C photoelectric conversion unit
20 horizontal signal reading circuit
21 amplification transistor
21D, 21S, 22D, 22S, 23S impurity area
21G, 22G, 23G gate electrode
21X, 22X, 23X gate insulating layer
22 reset transistor
23 address transistor
24 pixel
25 vertical scanning circuit
26 counter electrode signal line
27 vertical signal line
28 load circuit
29 column signal processing circuit
31 power supply wiring
32 differential amplifier
33 feedback line
34 charge storage node
35 charge detection circuit
36 address signal line
37 reset signal line
40 semiconductor substrate
41 device isolation region
50 interlayer insulating layer
51, 53, 54 contact plug
52 wiring
60 color filter
61 microlens
100 imaging apparatus

## Claims

1. A photoelectric conversion device comprising:
a first electrode;
a second electrode facing the first electrode; and
a photoelectric conversion layer located between the first electrode and the second electrode and including a bulk heterojunction layer containing a donor organic compound and an acceptor organic compound, wherein
the donor organic compound includes a first substituent, and
the acceptor organic compound includes an aromatic portion and a second substituent that binds to the aromatic portion and that has dipole-dipole interaction with the first substituent.

2. The photoelectric conversion device according to claim 1, wherein
the first substituent and the second substituent are each an alkoxy group, an alkylsulfanyl group, or a cyano group.

3. The photoelectric conversion device according to claim 1, wherein
the first substituent and the second substituent are each an alkoxy group or a cyano group.

4. The photoelectric conversion device according to any one of claims 1 to 3, wherein
the donor organic compound is a phthalocyanine derivative or a naphthalocyanine derivative.

5. The photoelectric conversion device according to claim 4, wherein
the donor organic compound is a phthalocyanine derivative represented by following formula (1) or a naphthalocyanine derivative represented by following formula (2): 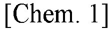 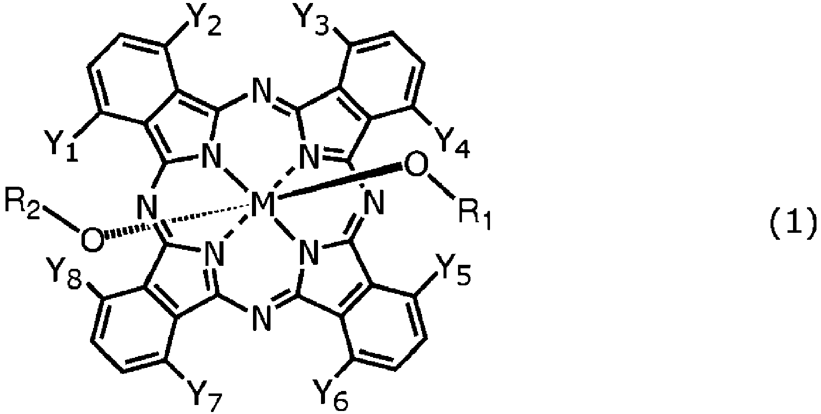 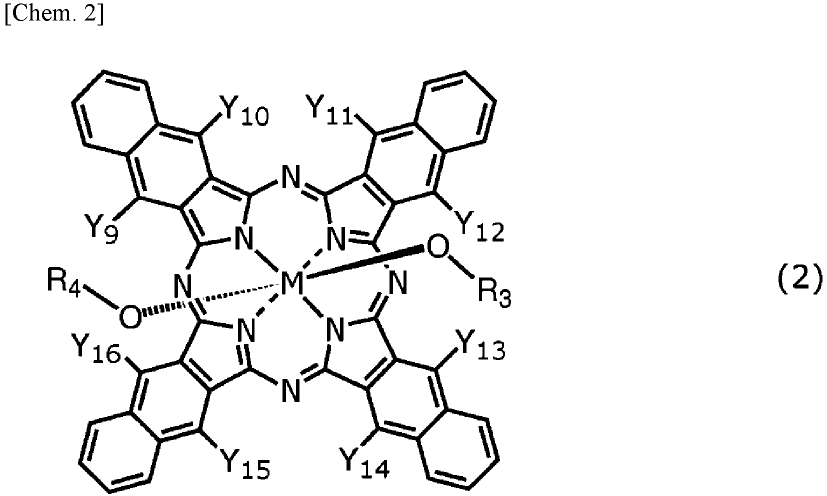 wherein, Y₁ to Y₁₆ are each independently the first substituent, M is Si, Sn, or Ge, R₁ to R₄ are each independently any one of substituents represented by following formulae (3) to (5), R₅ to R₇ are each independently an alkyl group or an aryl group, and R₈ to R₁₀ are each independently an aryl group,

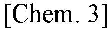

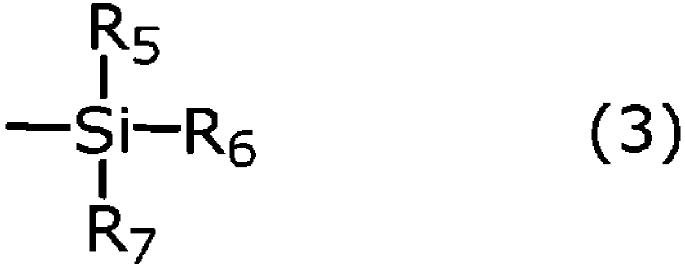

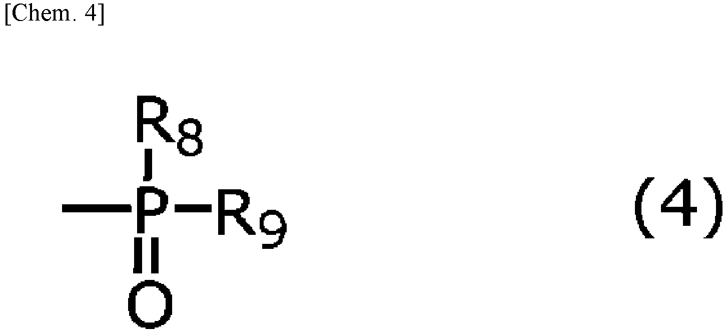

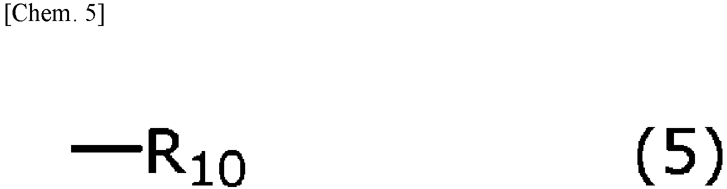

6. The photoelectric conversion device according to any one of claims 1 to 5, wherein
the acceptor organic compound is a fullerene derivative.

7. The photoelectric conversion device according to claim 6, wherein
the fullerene derivative is C60 fullerene bound to the second substituent or [6,6]-phenyl-C61-butyric acid methyl ester bound to the second substituent.

8. The photoelectric conversion device according to any one of claims 1 to 7, further comprising:
a buffer layer located between the photoelectric conversion layer and at least one selected from the group consisting of the first electrode and the second electrode.

9. An imaging apparatus comprising:
a substrate; and
a pixel including a charge detection circuit provided in the substrate, a photoelectric conversion unit provided on the substrate, and a charge storage node electrically connected to the charge detection circuit and the photoelectric conversion unit, wherein
the photoelectric conversion unit includes the photoelectric conversion device according to any one of claims 1 to 8.
